(19)

Europäisches
Patentamt
European
Patent Office
Office européen
des brevets

(11) **EP 4 668 276 A1**

(12) **EUROPEAN PATENT APPLICATION**

(43) Date of publication:
**24.12.2025  Bulletin 2025/52**

(21) Application number: **24183325.0**

(22) Date of filing: **20.06.2024**

(51) International Patent Classification (IPC):
*G16B 20/00* (2019.01)        *G16B 40/00* (2019.01)

(52) Cooperative Patent Classification (CPC):
**G16B 20/00; G16B 40/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **BASF Agricultural Solutions US LLC
Research Triangle Park, NC 27713 (US)**

(72) Inventors:
• **PITA, Fabiano V
  Durham, North Carolina 27709 (US)**
• **BONDALAPATI, Krishna
  Morrisville, North Carolina 27560 (US)**
• **COLMANT, Alexandre
  9052  Gent (BE)**
• **SUN, Xiaochun
  Morrisville, North Carolina 27560 (US)**

(74) Representative: **Maiwald GmbH
Elisenhof
Elisenstraße 3
80335 München (DE)**

(54) **METHOD FOR DECISION SUPPORT IN SELECTING CROSSINGS FOR POPULATION**

(57)    The invention provides a computer-implemented method for decision support in selecting crossings for population development, the method comprising:
receiving a set of candidate parents, a set of traits, and one or more constraints comprising a resource constraint representative of a maximum number of crossings and/or a maximum progeny population size; carrying out a multi-objective optimization; evaluating a pareto front of the set of solutions and selecting a subset of one or more solutions within the set of solutions based on a result of evaluating the pareto front; selecting a solution of the subset of solutions; and providing the list of crossings of the selected solution and a predicted required number of progenies for each of the crossings comprised in the list of crossings.

**Fig. 4**

**Description**

FIELD OF THE INVENTION

The invention provides a method for decision support in selecting crossings for population development, a system, a computer program product, and a computer-readable medium.

BACKGROUND

Plant breeding is the science of changing the traits of plants in order to produce desired characteristics which is used for cultivar development, crop improvement, and seed improvement. Plant breeding can be carried out by using different techniques including simply selecting plants with desirable characteristics for propagation, methods of using knowledge of genetics and chromosomes as well as complex molecular techniques. The modern plant breeding techniques include, for instance, marker assisted selection, genetic modification, genomic selection, and reverse breeding and doubled haploids. The goals of plant breeding are to produce crop varieties that boast unique and superior traits for a variety of applications. The interested traits include increased nutrition, flavor, and yield of crops, increased tolerance to environmental pressures (e.g. salinity, drought), increased tolerance to herbicides, and resistance to fungi, bacteria, and insect. Traditionally, breeding involves the creation of multi-generation genetically diverse populations on which human selection is practiced to generate adapted plants with new combinations of specific desirable traits. The selection process is driven by biological assessment in relevant target environments and available knowledge of genes and genomes. Progress is assessed based on gain under selection, which is a function of genetic variation, selection intensity, and time.

At present selecting crossings of parents, e.g. for plant population development, is very challenging. Generally, plant population development is done with the aim of increasing certain traits. However, it is very difficult to objectively decide which parents to select for crossing and how to pursue population development. In addition, the improved plant varieties should face the various biotic and abiotic stress factors that are increasing with the current climate changes. Moreover, the breeding and selecting can be very time-consuming. Even with the very latest in biotech-assisted conventional breeding, incorporation of a trait may take an average of seven generations for clonally propagated crops, nine for self-fertilizing, and seventeen for cross-pollinating.

Approaches for selecting crossings may be done by a person, e.g. a breeder, in some cases supported by statistical approaches. However, overall the selection process requires improvement. Particularly, there is a need for providing automatic decision support in selecting crossings for population development.

Thus, it is an object of the present invention to provide a method that allows for overcoming at least some of the above challenges, particularly providing a method and system for decision support in selecting crossings for population development.

SUMMARY

The object is achieved by the present invention. The invention provides methods, systems, computer program products, and computer-readable mediums according to the independent claims. Preferred embodiments are laid down in the dependent claims.

The present disclosure provides a computer-implemented method for decision support in selecting crossings for population development, the method comprising:

receiving a set of candidate parents, a set of traits, and one or more constraints comprising a resource constraint representative of a maximum number of crossings and/or a maximum progeny population size;

carrying out a multi-objective optimization,

wherein carrying out the optimization comprises, constrained by the received one or more constraints, optimizing at least a first optimization objective, a second optimization objective, and a third optimization objective, and outputting a set of solutions, wherein each solution is a list of crossings of parents (each crossing being a crossing of two parents) of the set of candidate parents,

wherein the first optimization objective is a short-term genetic gain objective associated with the set of traits and the optimization comprises maximizing the short-term genetic gain,

wherein the second optimization objective is a long-term genetic gain objective and the optimization comprises maximizing the long-term genetic gain, and

wherein the third optimization objective is a resource requirements objective associated with a predicted required number of progenies and the optimization comprises minimizing the resource requirements;

evaluating a pareto front of the set of solutions and selecting a subset of one or more solutions within the set of solutions based on a result of evaluating the pareto front.

outputting, to a user, a representation of the potential crossings, their associated short-term genetic gain and long-term genetic gain, and a representation of the subset of one or more solutions, and receiving a user input selecting a solution of the subset of solutions, or

automatically ranking the solutions of the subset of solutions based on an objective function used in the multi-objective optimization and automatically selecting the highest-ranked solution;

providing the list of crossings of the selected solution and a predicted required number of progenies for each of the crossings comprised in the list of crossings.

Thus, a method is provided that provides a list of crossings of parents, i.e., the selected solution, and, for each of the crossings a predicted required number of progenies. The method as disclosed herein allows for semi-automated or fully automated selection of the solution.

Based on the solution, breeding may be carried out to obtain respective progenies of the respective crossing of parents. This may be considered to be population development, the population development being based on the list of crossings.

This relates particularly to plant population development.

With conventional methods, it would take more generations and/or more crossings and associated resources to obtain similar results. Therefore, the present method allows for better resource allocation and accelerates population development. To some degree, the present method alleviates trial and error of existing methods. In addition, the method of the present disclosure allows for low demand in processing and computing resources.

As such, the above-mentioned challenges and other challenges are addressed by the method disclosed herein.

In other words, the present method comprises the above multi-objective optimization and subsequently carrying out a sequential selection process, wherein, in a first selection step, a subset of one or more solutions within the set of solutions may be is selected based on evaluation of the pareto front, e.g., by finding solutions at or near the pareto front and selecting them as subset. The solution(s) may be associated (and, in case of an intended subsequent user selection, presented together) with respective compromises of at least one of the optimization objectives. The first selection step may, for example, be fully automated.

In a second selection step, a solution may be selected among the subset of solutions. This may be done in different ways.

For example, the method may comprise automatically determining a ranking of the solutions based on an objective function used in the multi-objective optimization and automatically selecting the highest-ranked solution. This scenario may allow for full automation of the selection of a solution. Alternatively, receiving a user input may be involved in the second selection step. A representation of the potential crossings, their associated short-term genetic gain and long-term genetic gain, and a representation of the subset of one or more solutions may be outputted to a user. A user input selecting a solution of the subset of solutions may then be received. Thus, in the user-selection scenario, display at a graphical user interface may allow for the user to review the compromises. In both scenarios, after the subset selection, further evaluation and selection of the solution is based on quantitative analysis and potentially reporting, of the impact of such compromises on the objectives.

The second selection step may involve receiving user input, and, the method may comprise, after the first selection step, outputting, to a user, a representation of the potential crossings, their associated short-term genetic gain and long-term genetic gain, and a representation of the subset of one or more solutions, and the second selection step may comprise receiving a user input selecting the at least one solution of the subsets of solutions.

Optionally, as will be explained below, potential solutions of the subset of solutions may be checked as to whether the respective associated compromises are acceptable. Predetermined criteria may be applied to that end. Otherwise, the optimization and selection of a subset may be executed again with (minor) adjustments, e.g. adjusted constraints and/or objective in the optimization and/or adjusted evaluation of a pareto front, e.g. search parameters, and/or adjusted reviewed objectives in the selection in order to identify an optimal solution for the modified objectives.

It is to be understood, while above three optimization objectives are mentioned as an example, more than three optimization objectives may be used.

The optimization of the optimization objectives may be independent or dependent.

It is to be understood that, herein, "population", "parent", "progeny" and like terms refer to plants, particularly plant breeding. Thus, the methods herein are to be understood as not entailing animals or humans. The plant population shall mean the number of plants in an area of land. It is calculated by dividing the number of seeds planted by the number of acres. Plant populations exhibit genetic variability due to random mutation, gene flow, and natural selection. Progeny may refer to a genetic descendant or offspring, and to any generation plant. A progeny plant may be from any filial generation, e.g., F1.

In the present disclosure, the term "user" is to be understood broadly and can be any human providing input via a user interface. In general, the method may be a distributed method and user interaction or user input may involve different users, different times, different input devices, and the like. The term "user" may entail a software scientist and/or breeding scientist and/or a breeder. The method, as such, works irrespective of the user identity and role.

The resources, herein, may refer to resources associated with crossing parents to obtain the progeny, upkeep of the progeny, and study of the progeny. Resource constraints may be associated with a maximum number of potential parents and, accordingly, maximum number of possible crossings, and/or an overall maximum number of progenies. The number of progenies is referred to as the progeny population size, which can for example be a per-crossing and/or a total progeny population size. Thus, a resource constraint may be associated with a maximum progeny population size. In any case, it may be desirable to reduce the number of progenies to reduce resource requirements. This may be accomplished, in the present disclosure, by a corresponding optimization objective, the resource requirements objective, which may aim at minimizing the predicted required number of progenies.

In particular, a resource according to the present invention shall mean any factor which is necessary to accomplish a breeding goal or carry out a breeding activity. In short, they are the components to achieve breeding goal including tangible and intangible assets, but is not limited to those. The resource can be plants, plant materials, and plant germplasm used for breeding.

It will be understood from the detailed discussion below, that the method may comprise predicting the required number of progenies. This prediction may be genomics-based and usually involves statistical methods.

The short-term genetic gain may be an overall improvement in aggregated trait values over the course of one or more breeding generations, as an example. By ways of the short-term genetic gain objective, the method aims at maximizing the short-term genetic gain.

As will be discussed in more detail below, a long-term genetic gain may be indicative of a projected genetic diversity over multiple generations. By ways of the long-term genetic gain objective, the method aims at maximizing the long-term genetic gain.

Genetic gain or genetic gain from selection may be seen as the improvement in average genetic value in a population or the improvement in average phenotypic value due to selection within a population over cycles of breeding (Hazel and Lush, 1942).

The multi-objective optimization may be based on an objective function. A feasible solution that minimizes or maximizes (depending on the formulation of the optimization problem) the objective function may be considered an optimal solution. Usually there are several local minima/maxima, such that several solutions will be yielded, i.e., a set of solutions.

Evaluating the pareto front may involve determining the pareto front and optionally finding solutions at the pareto front and/or finding solutions within a certain distance from the pareto front. Thus, solutions at or near the pareto front may be selected. The term "near the pareto front" is to be understood as meaning "within a predetermined distance from the pareto front".

Ranking the solutions based on an objective function may, for example, rank solutions according to their respective scores on the scale of the objective function.

A crossing may be indicated, in the list, by the pair of parents to be crossed. As such, a list of crossings (i.e. a solution) may comprise a list of pairs of parents to be crossed.

The invention also provides a method comprising the methods for decision support of the present disclosure, and further comprising using the list of crossings and selecting and crossing the parents in the manner indicated in the list for generating a progeny population and/or providing resources for population development based on a/the list of crossings.

That is, a method may be provided that employs the selected solutions, specifically the provided selected list of crossings and the predicted required number of progenies for each of the crossings of said list of crossings.

Based thereon, breeding may be carried out to obtain respective progenies of the respective crossing of parents. This may be considered to be (breeding) population development based on the list of crossings. The respective resources for the population development may be provided, such as spatial resources, material resources, and time-related resources, the resources allowing for crossing the parents and then growing and studying the progenies.

With conventional methods, it would take more generations and/or more crossings and associated resources to obtain similar results. Therefore, the present method allows better resources allocation and allows for more efficient (plant) population development.

The invention also provides a system comprising a computing system configured to carry out the method of the present disclosure.

The invention also provides a computer program product comprising instructions which, when the program is executed by a computer, cause the computer to carry out the methods of the present disclosure.

The invention also provides a computer-readable medium comprising instructions which, when executed by a computer, cause the computer to carry out the methods of the present disclosure.

The features and advantages outlined above in the context of the method similarly apply to the system, the computer program product, and the computer-readable medium described herein.

According to the present disclosure, evaluating the pareto front comprises using an algorithm, such as a search algorithm, to select a subset of one or more solutions within the set of solutions.

In an example, a search algorithm may find and select a solution that scores best across all objective functions. In an example, if the constraints are too strict, a user may restart the search algorithm procedure with more relaxed constraints (or with less constraints).

The algorithm may be an algorithm that finds the pareto front and distance of the solutions from the pareto front. It may yield solutions at or within a predetermined distance from the pareto front. It is noted that the solutions will be associated with different trade-offs or compromises. The algorithm will narrow down the potential solution list based on the solution's relation to, e.g. distance from, the pareto front. Such a selection can be considered to be an objective selection that does not require to know and take into account the actual content of each solution or meaning/consequence of the tradeoff. This allows for ease of transfer to different scenarios and does not require availability/knowledge of all underlying information and principles.

The method according to the present disclosure may comprise, prior to carrying out the optimization, pre-selecting a subset of candidate parents among the set of candidate parents based on genomic information, wherein the optimization is carried out only for crossings of the subset of candidate parents.

As an example, the genomic information may comprise genomic breeding values, trait profile (e.g. native and transgenic traits), marker frequency, marker-based additive and/or dominance effects, and quantitative genetic loci (QTL) associated with traits of agronomic importance (disease resistance, flavor, shape, color, maturity, and others).

In other words, commonly used genomic breeding values or values derived therefrom can be employed to identify, in advance, the most suitable input data into the optimization, which reduces computational resources of the optimization and subsequent steps, and overall is likely to yield better results. It can be seen as a filter for input data that filters for specific genomic properties of the potential parents.

The method according to the present disclosure may particularly comprise simulating, making use of genomic information, mean and variance of a progeny for every possible crossing. The optimization may be carried out only for a subset of crossings for which feasibility for a set of genetic gain targets has been confirmed based on the simulated mean and variance of a progeny.

For example, only parents associated with said subset of crossings may be input into the optimization.

The mean and variance of a progeny may relate to any trait that shows a quantitative distribution of the observed genomic breeding values. Such traits can be assumed controlled by many genes with additive and/or dominance effects. These effects are specific to the considered breeding program and are derived using statistical methods known as genomic selection methods.

Mean and variance of a progeny may be predicted using simulation or prediction equations, either using pre-calculated marker effects that were previously calculated using common methods.

According to the present disclosure, the short-term genetic gain and/or the long-term genetic gain may be determined making use of (the) genomic information.

The method of the present disclosure may comprise determining the expected short-term genetic gain and/or long-term genetic gain. Known simulation methods, which are generally statistical methods, may be used for determining the genetic gains. Accordingly, the determining will generally be carried out automatically.

According to the present disclosure, the short-term genetic gain may be determined based on, e.g. stochastically, predicted superior progeny values for each of the crossings, in particular wherein the superior progeny value for the respective crossing is an aggregated value aggregated over the set of traits with optional weighting of the traits.

The superior progeny value may be considered a short-term genetic gain potential score of a given crossing combination. It may be calculated using known methods. Further details are also provided below.

The superior progeny value may be a value of a particular cross thus depends on the expected performance of its best progeny. Superior progeny value may be a linear combination of the mean of the cross's progeny and their standard deviation. A description and method for calculating the superior progeny value is, for instance, described in Shengqiang Zhong and Jean-Luc Jannink, Genetics 177, 567-576 (2007).

According to the present disclosure, the long-term genetic gain may be representative of a genetic diversity, wherein the genetic diversity is expressed by a minor allele frequency score and/or an inbreeding coefficient. Genetic diversity or long-term genetic gain may express an overall expected level of genotypic variance among individuals in a population.

The minor allele frequency score may be a score (e.g. a value) that quantifies a minor allele frequency. Specifically, the minor allele frequency score may identify the number of rare alleles in a population basis which a specific individual and its potential crossing combinations carry. Individuals with higher scores are desirable since they allow carrying forward rare alleles that can be valuable for future generations in the breeding program.

The inbreeding coefficient is a measure that quantifies how closely related individuals are in a population due to common ancestry. The higher this coefficient, the lesser is the genetic variability in a population. The inbreeding coefficient is well known to a person skilled in the art. The description of a method for calculating the inbreeding coefficient is, for instance, described in Deniz Akdemir and Julio I. Sanchez, Efficient Breeding by Genomic Mating, Frontiers in Genetics, Vol. 7, 210, (2016).

The above can be used as predictors for long-term genetic gain and allow to quantitatively take it into account in an automatic determination of solutions, particularly in an optimization.

According to the present disclosure, the inbreeding coefficient of a solution may depend on at least one of a number of times a parent is used for crossing in the solution, a number of unique parents used for crossing in the solution, and the similarity of the parents of the respective crossing.

According to the present disclosure, the long-term genetic gain objective may be based on only one of the inbreeding coefficient and the minor allele frequency score. This may already yield good results while reducing complexity and resource requirements of the determination of solutions.

According to the present disclosure, the long-term genetic gain objective may be based on both, the inbreeding coefficient and the minor allele frequency score. This may improve the overall results, i.e., improve the selected solution, particularly in terms of the long-term genetic gain. Optionally the method may comprise normalization of the inbreeding coefficient and the minor allele frequency score and combining the normalized inbreeding coefficient and minor allele frequency score into a single optimization objective - which may be called diversity measurement. This allows for making it easier and more efficient for the optimization to find a result.

The subset of one or more solutions may be a first subset of one or more solutions and the method according the present disclosure may comprise determining prior to selecting a solution of the subset of solutions, particularly in response to a user input and/or by automatic determination based on the optimization objective, whether any of the first subset of solutions meets predetermined criteria and, in case none of the first subset of solutions meets the predetermined criteria:

a) modifying the set of candidate parents, the set of traits, and/or at least one of the one or more constraints;

b) carrying out the optimization with the modified set of candidate parents, the modified set of traits, and/or the modified constraint(s), the optimization comprising outputting a second set of solutions;

c) evaluating, particularly using a search algorithm, a pareto front of the second set of solutions and selecting a second subset of one or more solutions within the second set of solutions based on a result of evaluating the pareto front;

d1) outputting, to a user, a representation of the potential crossings, their associated short-term genetic gain and long-term genetic gain, and a representation of the second subset of one or more solutions, and receiving a user input selecting a solution of the second subset of solutions or of the first and second subset of solutions, or

d2) automatically ranking the solutions of the second subset of solutions based on an objective function used in the multi-objective optimization and automatically selecting the highest-ranked solution among the second subset of solutions or among the first and second subset of solutions;

providing the list of crossings of the selected solution and a predicted required number of progenies for each of the crossings comprised in the list of crossings.

Optionally, the method may only go to steps d1) or d2) in case it is determined that the second subset of solutions meets the predetermined criteria, and otherwise the method may return to step a).

That is, an iterative process may be provided that allows for repeating optimization with adjusted settings in case the subset of solutions has no solution that is considered acceptable based on predetermined criteria. Solutions may not meet criteria due to the constraints or combination of constraints. For example, some constraints may be loosened or tightened selectively. Moreover, the selection of candidate parents may have been such that no sufficiently good results could be yielded, e.g. due to the parent's trait profile. In this case using a different set of candidate parents may improve the results. Also the set of traits, which are in some cases conflicting, may be a reason for not obtaining sufficiently good solutions. Thus, the set of traits may be changed.

The meeting of criteria may be determined automatically. Alternatively or in addition, a user may evaluate whether criteria are met. User input may be received, which may trigger automatic modifying or provide input for the modifying of step a). The modifying may be done based on received user input and/or based on automatic adherence to rules for modifying. Said rules may be based on theoretical considerations, or be empirically or semi-empirically determined rules. Also, the modification can be obtained using artificial intelligence algorithms, e.g. artificial intelligence algorithms that can extensively evaluate the larger pool of candidates available in a breeding program.

Optionally, after the first repetition of the optimization, the method may proceed with the (final) selection of a solution, irrespective of whether criteria are met. Alternatively, repetitions may continue until the criteria are met, optionally unless a predetermined number of repetitions has been reached before the criteria are met.

As an example, solutions with maximum acceptable compromises (in different aspects) may be used as benchmarks for determining whether criteria are met. For example, maximum (acceptable) liability and minimum (acceptable) benefit can be expressed in the form of quantitative criteria that can be applied to the solutions. If no solution meets those criteria, they should not be pursued, which can be ensured by the above iterative process.

According to the present disclosure, the constraints may comprise constraints related to the inclusion and/or exclusion of specific crosses and/or constraints related to achieving a maximum value for one or more predetermined traits, and/or constraints related to achieving a constant value for one or more predetermined traits, and/or constraints related to achieving a minimum value for one or more predetermined traits, and/or constraints related to a predetermined proportion of crosses with a desirable characteristic that should make up the final crossing list.

The one or more constraints may comprise at least one of a minimum short-term genetic gain, a minimum solution size, a maximum solution size, an inclusion constraint associated with one or more crossings to be included in each of the solutions, a constraint associated with one or more crossings to be included or excluded from each of the solutions, a secondary traits constraint associated with one or more secondary traits.

Constraints related to the inclusion and/or exclusion of specific crosses may be seen as constraints related to must-have/may-not-have specific crosses. The desirable characteristics may comprise target crossing outcomes for categorical traits (such as disease resistance, etc.).

According to the present disclosure, the subset of solutions may comprise solutions at or within a predetermined distance from the pareto (dominant) front of solutions of the multi-objective optimization.

That is, the pareto front of solutions may be determined and a predetermined distance, e.g., fixedly set or selected by a user may be compared to an actual distance of each of the solutions from the pareto front. Only if the solution is at the pareto front or if the actual distance is equal to or smaller than the predetermined distance, the solution may be selected to be part of the subset of solutions.

This allows for an objective selection that yields good results. If allowing for a predetermined distance from the pareto front, it is at the same time possible to reduce the likelihood of a very small or zero subset of solutions, which could result from, e.g., only selecting solutions exactly at the pareto front.

According to the present disclosure, the representation of the potential crossings and their associated short-term genetic gain and long-term genetic gain may comprise representing each crossing within a two-dimensional coordinate system, the coordinate system having a first axis representative of the short-term genetic gain and a second axis representative of a long-term genetic gain.

As such, information required for making a selection of a solution with all relevant information, particularly taking into account trade-offs, can be made, which improves decision accuracy and outcome.

Variation of the predetermined distance may allow for tuning the subset size in a quantitative manner related to quality of the solutions.

According to the present disclosure, the representation of the subset of solutions comprises, for each solution of the subset, an indication of a group of crossings associated with the solutions, particularly the group being represented in the coordinate system such as by heatmaps or shapes (in other words: symbols, indication, representations, for example boxes) enclosing the crossings of the group of crossings or other highlights highlighting crossings of the group of crossings.

The shapes or other highlights allow for providing all information required to evaluate the tradeoffs made by selecting one of the solutions. That is, a user will be provided with the information, which crossings belong to which solution while still being able to review the quality (e.g. tradeoffs) of each crossing, and thereby being able to make the most informed decision and selection among the solutions.

The shapes enclosing the crossings may be of any shape. For example, shapes and/or colors of the shapes enclosing the crossings may be employed to distinguish between solutions. Other representations/highlights are also conceivable, but the shapes may allow for little interference with the remaining areas, i.e., reduced information loss.

According to the present disclosure, optionally, the representation of the potential crossings, and e.g. their associated diversity, may further comprise representing each crossing as a geometric shape, such as a circle, within the coordinate system, the size of the geometric shape being correlated with a first metric of the long-term genetic gain and the second axis value is correlated with a second metric of the long-term genetic gain, in particular wherein the first metric is associated with one of an inbreeding coefficient and a minor allele score, and the second metric is associated with the other one of the inbreeding coefficient and the minor allele score.

Such representation allows for providing all information (which is multi-dimensional) that is required to select solutions based on trade-offs in one representation, which improves selection accuracy and reduces trial and error compared to other representations that fail to provide all information concurrently.

TERMINOLOGY

"As used herein, terms in the singular and the singular forms like "a", "an" and "the" include plural referents unless the content clearly dictates otherwise. Thus, for example, reference to "plant", "the plant" or "a plant" also includes a plurality of plants; also, depending on the context, use of the term "plant" can also include genetically similar or identical progeny of that plant or plants derived therefrom by crossing. Also as used herein, the word "comprising" or variations such as "comprises" or "comprising" will be understood to imply the inclusion of a stated element, integer or step, or group of elements, integers or steps, but not the exclusion of any other element, integer or step, or group of elements, integers or steps.

As used herein, the term "and/or" refers to and encompasses any and all possible combinations of one or more of the associated listed items, as well as the lack of combinations when interpreted in the alternative ("or"). The term "comprising" also encompasses the term "consisting of".

Below, some terminology will be provided. The terminology is provided for illustrative purposes, and particularly for understanding the subsequent detailed examples. The terminology is not to be understood as limiting to the claim scope.

Related to combinatorial optimization:

- Solution/alternative: A specific and unique outcome to a decision making problem

- Decision variables: A set of unknowns which, when assigned specific known values from a finite set, combinedly depict the constitution of a particular solution

- Objective function: A metric used to measure the desirability of a solution with respect to a certain criterion

- Hard constraint: A condition used to assess whether or not a solution is realistically and practically implementable (i.e. a condition used to assess the feasibility of a given solution)

- Soft constraint: A condition on the ideal constitution of solutions, the violation thereof incurs certain penalty costs in the/one-of-the objective function(s)

- Optimization: The process of finding the most desirable/preferred feasible solution in a decision making problem

- Combinatorial optimization problem: A decision making problem with a finite amount of solutions in which the desirability and feasibility of each solution can be measured and assessed

- Optimization model: The abstract blueprints of an optimization problem, used to map decision-making goals to computational programming code.

- Move: A transformation used to slightly alter the inherent composition of a given solution

- Neighbor solution: A solution that can be generated from any other given solution after performing a single move

- Solution neighborhood: The set of all potential neighbor solutions to a given solution

- Search algorithm: A finite sequence of moves used to find one or more feasible desirable solutions in an optimization problem

- Initial solution: The starting point of a search algorithm.

- Domain space: The set of all solutions

- Feasible domain space: The set of all feasible solutions

- Objective space: The set of all objective function(s) measurements forming a one-to-one or one-to-many relationship with all solutions in the domain space

- Search space: The set of all potential solutions in the radar of a search algorithm

- Constraint relaxation: The act of reducing the impact of a hard constraint on the feasible domain space as a means to improve the navigation capabilities of the search algorithm throughout the search space.

- Dominance: A condition used to assess whether a given solution scores better than another given solution across all objective functions in a multi-objective optimization problem

- Archive: A dynamic set keeping track of all feasible non-dominated solutions found during the course of a search algorithm

- Non-dominated front: The final archive obtained, upon termination of the search algorithm

- Pareto front: The best possible non-dominated front that intrinsically exists (but that has no certainty of being uncovered).

Related to plant breeding:

- Scenario: A plant breeding program categorized by type of crop, breeding season, and geographical location on the globe

- Breeding population: A population of plant individuals of a given crop in the experimental phase of a scenario

- Crossing combination: A specific pair of plant individuals from the same cultivar to be used as potential parents breeding material

- Progeny: A child individual(s) obtained from the breeding process of a crossing combination

- Progeny population size: The total number of progenies bred from a specific crossing combination.

- Genotype: The genetic code of a plant individual

- Phenotype: The observable physical characteristics of a plant individual

- Trait: A phenotypic characteristic of interest to the breeders, farmers, decision makers and/or market

- Short-term genetic gain: The overall improvement in aggregated trait values over the course of generations

- Elite progeny: A progeny individual that meets or exceeds the targeted short-term genetic gain benchmark

- Superior progeny value: The estimated short-term genetic gain potential score of a given crossing combination

- Genetic diversity: The overall level of genotypic variance among the individuals in a cultivar. This will sometimes also be referred to as the long-term genetic gain.

- Primary trait: A trait whose attributes feature in the short-term genetic gain objective function in the optimization model

- Secondary trait: A trait whose attributes feature in a hard constraint in the optimization model

- Inbreeding: The process of breeding plant individuals that are closely genetically related.

- Cultivar: In general, a cultivar may be a kind of cultivated plant that people have selected for desired traits and which retains those traits when propagated. It may be an organism and especially one of an agricultural or horticultural variety or strain originating and persistent under cultivation. Cultivars are usually produced through artificial selection and are different forms of the same species. In the present disclosure, cultivar can have equal meaning of breeding population and selected crossings.

Further features, examples, and advantages will become apparent from the detailed description making reference to the accompanying drawings.

BRIEF DESCRIPTION OF THE DRAWINGS

In the accompanying drawings,

Fig. 1                illustrates an exemplary method according to the present disclosure;

Fig. 2                illustrates another exemplary method according to the present disclosure;

Fig. 3                illustrates another exemplary method according to the present disclosure;

Fig. 4                illustrates a system according to the present disclosure;

Fig. 5                illustrates another exemplary method according to the present disclosure;

Figs. 6a and 6b    illustrate statistical considerations on population size and genetic gain;

Figs. 7a to 7c      illustrate exemplary visualizations of example solutions;

Fig. 8                illustrates simulation results of an evolution of a breeding population relative to their overall phenotype performance;

Figs. 9 and 10      illustrate exemplary secondary traits constraints input data.

DETAILED DESCRIPTION

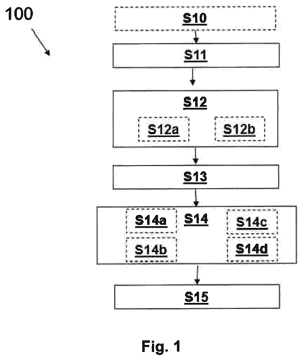

Fig. 1 illustrates an exemplary computer-implemented method **100** for decision support in selecting crossings for population development according to the present disclosure.
In step **S11**, the method comprises receiving a set of candidate parents, a set of traits, and one or more constraints comprising a resource constraint representative of a maximum number of crossings and/or a maximum progeny population size.
In other words, an input may be provided that specifies which parents are candidates for potential crossings. Furthermore, a desired set of traits is provided as input. Constraints for the optimization may be provided as input, which comprise the resource constraints. Resource constraints will generally comprise a maximum number of crossings and/or maximum progeny population size, i.e., a maximum number of progenies.
In step **S12**, the method comprises carrying out a multi-objective optimization.
Carrying out the optimization comprises, constrained by the received one or more constraints, optimizing, in step **S12a**, at least a first optimization objective, a second optimization objective, and a third optimization objective, and outputting, in step **S12b**, a set of solutions, wherein each solution is a list of crossings (each crossing being a crossing of two parents) of the set of candidate parents. Optionally, the optimization objectives may be optimized independently. The use of three optimization objectives is merely illustrative. More optimization objectives may be employed.
The first optimization objective is a short-term genetic gain objective associated with the set of traits and the optimization comprises maximizing the short-term genetic gain. The second optimization objective is a long-term genetic gain objective and the optimization comprises maximizing the long-term genetic gain. The third optimization objective is a resource requirements objective associated with a predicted required number of progenies and the optimization comprises minimizing the resource requirements.
In other words, the optimization will be carried out and an objective function may be used to that end. Multiple solutions may be output by the optimization. Each solution is a list of crossings. The list of crossings may comprise, for each list entry, the two parents to be crossed. As will be seen below, the overall output of the method will yield, for each pair of parents/each crossing, how many progenies would probably be required, e.g., according to statistical predictions.
In step **S13**, the method comprises evaluating a pareto front of the set of solutions and selecting a subset of one or more solutions within the set of solutions based on a result of evaluating the pareto front.
As an example, the subset of solutions may comprise solutions at or within a predetermined distance from the pareto front of solutions of the multi-objective optimization.

Evaluating the pareto front may comprise using an algorithm, such as a search algorithm, to select a subset of one or more solutions within the set of solutions.

In step **S14** a solution of the subset of solutions is selected, particularly automatically or by receiving a user input.

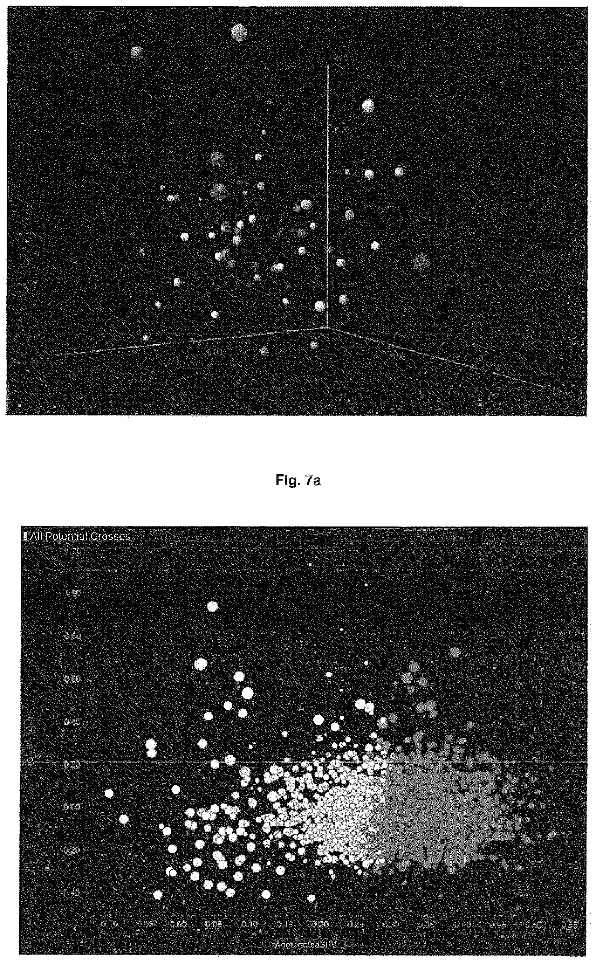

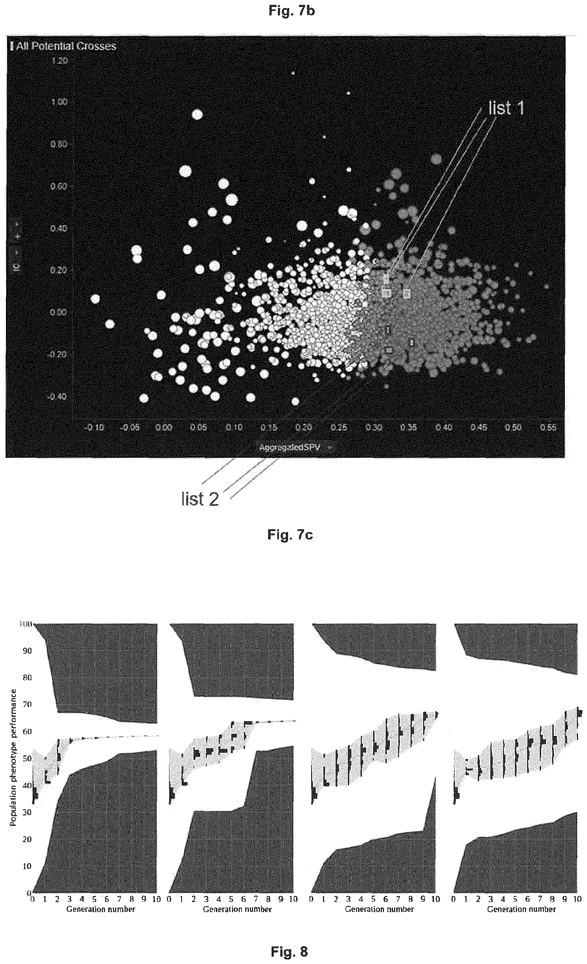

Specifically, the method may comprise, in step **S14a**, outputting, to a user, a representation of the potential crossings, their associated short-term genetic gain and long-term genetic gain, and a representation of the subset of one or more solutions, and receiving, in step **S14b**, a user input selecting a solution of the subset of solutions. Figs. 7b and 7c illustrate how solutions might be represented to allow an informed user selection.

Alternatively, the method may comprise, in step **S14c**, automatically ranking the solutions of the subset of solutions based on an objective function used in the multi-objective optimization and, in step **S14d**, automatically selecting the highest-ranked solution.

The method may comprise, in step **S15**, providing the list of crossings of the selected solution and a predicted required number of progenies for each of the crossings comprised in the list of crossings. The list may, among other uses, also be output to a user via a display device. The list may then be used for population development as described below.

In an optional step **S10**, prior to step **S11**, i.e., prior to carrying out the optimization, the method may comprise pre-selecting a subset of candidate parents among the set of candidate parents based on genomic information, wherein the optimization is carried out only for crossings of the subset of candidate parents. In particular, the method may comprise simulating, making use of genomic information, mean and variance of a progeny for every possible crossing. The optimization may be carried out only for a subset of crossings for which feasibility for a set of genetic gain targets has been confirmed based on the simulated mean and variance.

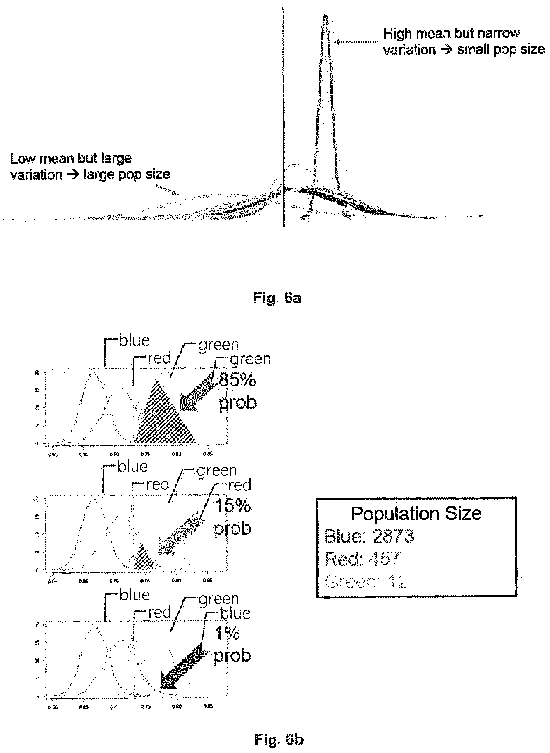

In other words, prior to carrying out optimization, the potential input into the optimization may be reduced by pre-selecting candidate parents using genomic information. As will be explained below, e.g. in the context of Figs 6a and 6b, this may be done using probabilities/statistics.

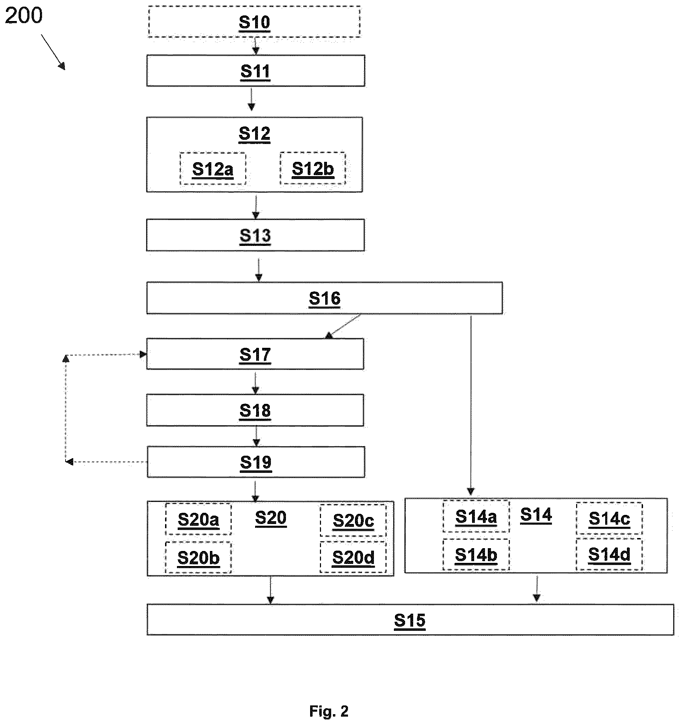

Fig. 2 shows another computer-implemented method **200** for decision support in selecting crossings for population development according to the present disclosure. Said method may be considered as having an iterative nature.

The steps **S11** to **S13** (and optionally **S10**) may the same as in Fig. 1 and reference is made to the above description of said steps. This also applies to steps **S14** and **S15**. The subset of one or more solutions is a first subset of one or more solutions.

In the present example, prior to selecting a solution of the subset of solutions (such as explained in the context of Fig. 1 in step **S14**) it is determined, in step **S16**, whether any of the first subset of solutions meets predetermined criteria. If this is the case, the method proceeds with step **S14** as explained in the context of Fig. 1. In case none of the first subset of solutions meets the predetermined criteria, the method proceeds with step **S17.**

In step **S17** (step a) in the claims), the method comprises modifying the set of candidate parents, the set of traits, and/or at least one of the one or more constraints.

In subsequent step **S18** (step b) in the claims), the method comprises carrying out the optimization with the modified set of candidate parents, the modified set of traits, and/or the modified constraint(s), the optimization comprising outputting a second set of solutions. This step may be carried out in the manner outlined in the context of Fig. 1 for step **S12.**

In subsequent step **S19** (step c) in the claims), the method comprises evaluating, particularly using a search algorithm, a pareto front of the second set of solutions and selecting a second subset of one or more solutions within the second set of solutions based on a result of evaluating the pareto front. This step may be carried out in the manner outlined in the context of Fig. 1 for step **S13.**

In subsequent step **S20a** (step d1) in the claims), the method comprises outputting, to a user, a representation of the potential crossings, their associated short-term genetic gain and long-term genetic gain, and a representation of the second subset of one or more solutions, and receiving, in step **S20b**, a user input selecting a solution of the second subset of solutions or of the first and second subset of solutions. This step may be carried out in the manner outlined in the context of Fig. 1 for steps **S14a** and **S14b.**

Alternatively, in step **S20c** (step d2) in the claims), the method comprises automatically ranking the solutions of the second subset of solutions based on an objective function used in the multi-objective optimization and automatically selecting, in step **S20d**, the highest-ranked solution among the second subset of solutions or among the first and second subset of solutions. This step may be carried out in the manner outlined in the context of Fig. 1 for steps **S14c** and **S14d.**

The method may then proceed to step **S15** of providing the list of crossings of the selected solution and a predicted required number of progenies for each of the crossings comprised in the list of crossings.

Optionally, the method may only proceed to step **S20a or S20c** only in case it is determined that the second subset of solutions meets the predetermined criteria in the manner described above in the context of step **S16** and otherwise returning to step **S17** (step a) in the claims). This may be repeated until the predetermined criteria are met, as indicated by the dashed arrows in Fig. 2.

Determining whether the solutions meet predetermined criteria may be in response to a user input and/or by automatic determination based on the optimization objective.

The method as shown in Fig. 2 and outlined above allows for further reducing trial and error in the selection of the solution and, thus, saves resources. Moreover, it is even less error prone than the method as described in Fig. 1.

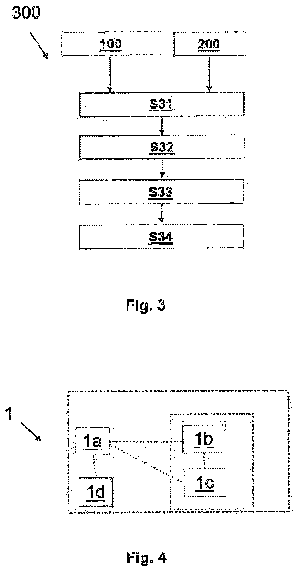

Fig. 3 illustrates a method that comprises a computer-implemented method **300** for decision support in selecting crossings for population development according to the present disclosure, which method may, for example, be or comprise the method **100** or **200** outlined above, and further comprises using, in step **S31**, the list of crossings and selecting, in step **S32**, and crossing, in step **S33**, the parents in the manner indicated in the list for generating a progeny population and/or providing, in step **S34**, resources for population development based on the list of crossings.

A system **1** according to the present disclosure is schematically illustrated in Fig. 4. Such a system may be employed in any of the methods according to the present disclosure, particularly one of the methods described in the context of Figs. 1 to 3. The system comprises a computing system **1a** configured to carry out the method of the present disclosure, particularly all automatic steps thereof. The computing system may be a distributed computing system or a non-distributed computing system, for example. The system may further comprise a display device **1b** and a user input device **1c**, which may be incorporated in the display device or separate from the display device. When the method comprises outputting a representation of potential crossings to a user, this may be done via the display device. When the method comprises receiving a user input, such as for selecting a solution, this may be done via the user input device. The list of crossings provided by the method may, for example, be displayed on the display device. Alternatively or in addition, it may be stored in an optional storage device **1d** or transmitted to another computing system. It is noted that one or more of elements **1b** to **1d** may be part of the computing system **1a.**

Further features, explanations and advantages associated with the method and system of the present disclosure will be outlined below.

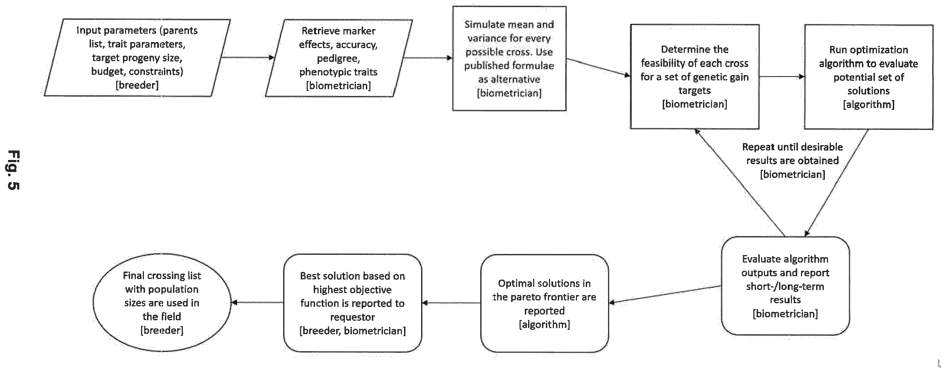

An overview of an exemplary method according to the present disclosure is illustrated in Fig. 5.

Input parameters (parents list, trait parameters, target progeny size, budget, constraints) may be provided. This may optionally involve user input, such as by a breeder.

Then, marker effects, accuracy, pedigree, phenotypic traits are retrieved. This may involve user input, such as by a biometrician.

Next, mean and variance for every possible cross can be simulated. Published formulae maybe used as alternative. This may optionally involve user input, such as by a biometrician.

Next, the feasibility of each cross for a set of genetic gain targets may be determined. This may optionally involve user input such as by a biometrician.

Then an optimization algorithm to evaluate potential set of solutions is carried out.

The steps of determining feasibility and carrying out an optimization algorithm may be carried out repeatedly until acceptable results are obtained. This might be determined automatically and/or involve input of a user, such as a biometrician.

An evaluation algorithm outputs and reports short-/long-term results, e.g. to a user such as a biometrician.

Optimal solutions in the pareto frontier are determined automatically, e.g. by an algorithm, and reported. This yields the subset of solutions.

A best solution may be selected, for example based on highest objective function, and may be reported to requestor, which may, for example, be a breeder or biometrician.

A final crossing list with population sizes corresponding to the solution may be output and used in the field, e.g. by a breeder.

As will be understood from the above, the choice of parents in breeding is crucial decision in a breeding program. If one is able to predict superior crosses, this allows gains in efficiency. These are concepts associated with breeding. The present disclosure allows to also take into account genomics, such as marker information permits to predict genetic variability within a population. While this allows a wealth of information there is still a huge space of possibilities for crossings and there may be many, in part conflicting parameters that need to be considered, which, in the present case, is tackled by optimization and subsequent selection steps. As such, the present method brings together breeding, genomics, and optimization to address the above-identified challenges.

In the following, some considerations on the optimization algorithm will be presented.

Figs. 6a to 6b illustrate statistical considerations on population size and genetic gain, which may, among others, be employed for preselection of parents in step **S10** above. The statistical probability to generate a superior progeny from a cross can be determined by calculating the area under the curve described by the predicted mean and variance of such a cross. This also indicates the feasibility of a potential cross to contribute to the targeted short term genetic gain. In specific, Figure 6a shows the typical differences among different crosses for the mean and variance predictions. Figure 6b shows that the probability of a progeny population to meet the short term genetic gain target can be translated into the size of such population in order to achieve this target with high confidence.

It is an aim of the present disclosure to obtain crossing lists, supported by the selection support tool, that meet: Multi-trait goals (short-term); Diversity (long-term); Resource Requirements (e.g. available resources); a biologically feasible genetic gain objective.

Known approaches can provide an expected mean of a progeny from a given cross. In addition, genomics can provide the expected distribution (variance) from a given cross (Figure 6a). Further details are provided below, for example in section 1.1.

Determining the number of progenies needed to have a certain confidence that at least a certain number of progenies meet a predetermined threshold is based on probabilities (Figure 6b). Further details are provided below, for example in section 1.2.

It is noted that the list of potential bi-parental crosses is extremely high and increases significantly when increasing the number of potential crossing partners even by small numbers. Thus, it is important to make a selection, but it is also difficult to obtain the best list a priori, particularly when trying to meet many conflicting objectives, such as yield, strength, diversity, resistance to environmental factors, resources (e.g. required for the actual progenies).

As an example, in the optimization stage, yield may be maximized, yield and diversity may be maximized (as such), and yield and diversity may be maximized *for a given number of progenies* that can be used.

Long-term genetic gain, e.g. involves increased diversity in the crossing block, e.g., minimizing an inbreeding metric or maximizing a rare allele score.

An example with numbers is provided below and illustrated in Figs. 7a to 7c.

In this example, 65 parents are used, which means there are 2080 potential crosses.

6 Traits are considered: Yield, Lint, Length, Strength, Mic, SFI. All traits are considered to have equal importance.

The aim is to maximize Yield, Lint, Length, Strength and to minimize SFI, and to keep MIC constant.

Some constraints/aims are selected as input, e.g. constraints concerning resources, e.g., the constraint of at most 15 entries for each population and at most 60 crosses in total. A genetic gain target is provided, e.g. a genetic gain target - 10% and 20% above the current NL-3 mean. A target is provided to obtain at least 3 progenies for each cross.

Fig. 7a illustrates the genetic relationship among candidate parents. This provides insights on the potential use of all parents or a subset of them for the objectives above. In general, very similar individuals (circles located close to each other in the graphic space) are redundant and only one individual should be used to represent that neighborhood (space in the graph). The whole space in the graph represents the whole available genetic diversity provided by the candidate parents.

Results illustrating short-term and long-term gain of potential crossings are illustrated in Fig. 7b. In this example, the following numbers are yielded and illustrated as explained below:

- 2080 crosses →

    - 1291 - 10% genetic gain

    - 871 - 20% genetic gain

- Short-term genetic gain

    - SPV → X-axis (maximize)

- Long-term genetic gain

    - IC → Y-axis (minimize)

    - RAF → bubble size (maximize)

Fig. 7c illustrates selection of two different lists of potential crosses (subset of solutions, each list being a solution).

Each crossing list will have trade-offs, while meeting general objectives. A person or an algorithm may select a final list, i.e., the solution or list to be output. If none of the solutions are considered adequate, the method may return to setting the constraints/aims that are selected and input (outlined above) to obtain a different set of solutions.

A potential solution, i.e. crossing list alongside the predicted number or progenies for each crossing (i.e., a potential output of the method of the present disclosure), is depicted in the table below, e.g., as obtained from the above example. It shows the solution ID, the parents, and the population size for each crossing, all output from the method of the present disclosure.

| ID | GGain | SolutionID | parent1 | parent2 | PopSize |
|---|---|---|---|---|---|
| 15_1 | Gain_15 | 1 | 15Q4I0098 | 16Q4I0149 | 9 |
| 15_1 | Gain_15 | 1 | 15Q4I0098 | 16Q4I0216 | 6 |
| 15_1 | Gain_15 | 1 | 15Q4I0098 | 16Q4I0232 | 5 |
| 15_1 | Gain_15 | 1 | 16Q4I0216 | 16Q4I0232 | 9 |
| 15_1 | Gain_15 | 1 | 15Q4I0098 | 16Q4I0269 | 7 |
| 15_1 | Gain_15 | 1 | 16Q4I0232 | EB 284141 | 8 |
| 15_1 | Gain_15 | 1 | 15Q4I0098 | EB 284141 | 5 |
| 15_1 | Gain_15 | 1 | 16Q4I0232 | EB 324410 | 5 |
| 15_1 | Gain_15 | 1 | 16Q4I0195 | EB 324410 | 10 |
| 15_1 | Gain_15 | 1 | 16Q4I0216 | EB 324410 | 7 |
| 15_1 | Gain_15 | 1 | 16Q4I0149 | EB 324410 | 10 |
| 15_1 | Gain_15 | 1 | EB 284141 | EB 324410 | 4 |
| 15_1 | Gain_15 | 1 | 16Q4I0232 | EB 3274 | 6 |
| 15_1 | Gain_15 | 1 | EB 284141 | EB 3274 | 5 |
| 15_1 | Gain_15 | 1 | EB 324410 | EB 3274 | 3 |
| 15_1 | Gain_15 | 1 | 15Q4I0098 | EB 3274 | 3 |
| 15_1 | Gain_15 | 1 | 16Q4I0232 | EB 3432 | 8 |
| 15_1 | Gain_15 | 1 | EB 324410 | EB 3432 | 5 |
| 15_1 | Gain_15 | 1 | EB 3274 | EB 3552 | 5 |
| 15_1 | Gain_15 | 1 | 16Q4I0216 | EB 3552 | 14 |
| 15_1 | Gain_15 | 1 | EB 3274 | EB 3813 | 5 |
| 15_1 | Gain_15 | 1 | 16Q4I0269 | EB 3813 | 14 |
| 15_1 | Gain_15 | 1 | EB 3432 | EB 3813 | 7 |
| 15_1 | Gain_15 | 1 | EB 3274 | EB 5928 | 3 |
| 15_1 | Gain_15 | 1 | 15Q4I0098 | EB 5928 | 3 |
| 15_1 | Gain_15 | 1 | EB 324410 | EB 5928 | 3 |
| 15_1 | Gain_15 | 1 | 16Q4I0232 | EB 5928 | 6 |
| 15_1 | Gain_15 | 1 | EB 3813 | FM 910 | 4 |
| 15_1 | Gain_15 | 1 | EB 284141 | FM 910 | 6 |
| 15_1 | Gain_15 | 1 | 16Q4I0232 | FM 910 | 6 |
| 15_1 | Gain_15 | 1 | EB 5928 | FM 910 | 3 |
| 15_1 | Gain_15 | 1 | 16Q4I0114 | FM 910 | 9 |
| 15_1 | Gain_15 | 1 | 15Q4I0098 | NM970123 | 10 |
| 15_1 | Gain_15 | 1 | EB 324410 | NM970123 | 10 |

Further examples and embodiments are outlined below.

**Initial considerations**

For thousands of years, farmers have adopted a methodological process toward the selection of agricultural plants with particular desirable characteristics (e.g. nutrition constituents, yield, shape, tolerance to environmental shifts, resistance to disease and herbicides, or increased storage/shelf life), also known as traits. Ideally, such plant specimens, when employed as progenitors for subsequent generations, result in the accumulation of desirable traits over time.

Today, plant populations are typically developed for the purpose of direct market release or as candidate parents in upcoming generations. Prior to the start of an agricultural phase, farmers are then typically required to order a selection of seeds to be planted on their fields. These seeds are developed and improved by plant breeding experts through the creation, management and evolution of various plant breeding programs known as cultivars.

Plant breeding is a rather complicated matter. On the one side, it would take a very long time for plant populations to improve significantly across multiple traits by means of mere trial-and-error approaches while, on the other side, observable trait attributes are not only induced by the genomic configuration of the plant itself, but also by the environment in which the plant is grown, and the interaction of the genotype with the environment. Significant crop improvement goals generally may be difficult to achieve without the combined expertise of research scientists and universities, large investment funds, information sharing, big data analysis, stochastic modelling and computerized optimization.

It is a goal to allow for deciding how the next population of a given crop should be developed from an (available) initial population. Uses of an advanced semi-automated software engine herein referred to as the Genomic Assisted Population Development (GAPD) is proposed. A purpose of this software is to assist breeders, farmers, and other various human decision makers, in making better decisions in the context of a plant breeding program.

The underlying process of the GAPD according to the present disclosure can be broken down into three broad steps: Firstly, raw input data are fed to the blueprints of a stochastic multi-objective combinatorial optimization model. Secondly, a multi-objective solution search metaheuristic, capable of extracting high-quality compromise solutions residing within the feasible domain space underlined by this mathematical model, is launched to explore the decision space delimited by this model. Lastly, the decision maker(s) may pick the most preferred compromise solution returned by this metaheuristic or an automated selection of a solution may be carried out, following which the corresponding solution may be communicated to the breeders and implemented.

## 1. Decision making goals

Decision making problems gravitate around the attainment of pre-established goals. While there always exists room for uncertainty and potential errors in decision making, there is perhaps no bigger source of inefficiency than the inability of decision makers to identify the fundamental goals of the underlying problem. Not only does that lead to inaccurate/inefficient output results, it can also create long setbacks for all those involved in the decision making problem. In this section the aim is to provide a description of the fundamental goals featuring in the GAPD.

### 1.1 On progeny population sizes and limited resources

Although progeny individuals inherit a great deal of their genotypic and phenotypic characteristics from their respective parents, due to factors such as random mutations and environmental conditions, the a priori knowledge of progeny phenotype attributes are still far from deterministic. Despite great advances in the fields of genomic and prototypic prediction, such attributes can therefore only be defined stochastically.

While progeny candidates share characteristics from both parents, the underlying target genotype can only be achieved if it actually manifests itself among the offspring population. Many studies hence suggest that sufficient amount of crosses should be conducted in order so that at least one progeny meets or exceeds these genetic gain targets with a certain probability. Practically speaking, plant breeders want to be able to increase the chances that a promising crossing combination will, a posteriori, produce at least a certain number of elite progeny individuals. Such elite individuals would then qualify as candidates to be released to the market and/or to become members of the subsequent populations. The number of progenies to be produced from a chosen crossing combination is referred to as the progeny population size.

One should note that there can certainly be other key reasons to produce multiple progenies from the same crossing combination, such as the death or malformation of progenies before they reach their full phenotype transformation state, or the opportunity to select other (relatively less important) trait characteristics not taken into account in the optimization model.

The calculation of crossing combinations population sizes is not particularly trivial. Out of the provided multi-dimensional Gaussian distribution of any given crossing combination (see also Figure 6a), the probability that a progeny meets the required genetic gain target is calculated by measuring the geometric hypervolume that falls beyond the multi-dimensional linear threshold induced through the specified target genetic gain. Theoretically speaking, this hypervolume is ideally evaluated with the use of definite integrals.

This multi-dimensional genetic gain threshold is constructed on a so-called global frame of reference. Upon these frames, the crossing combination probability distributions are fitted independently from one another by employing so-called local frames of reference. With the calculation of every crossing combination population size, the corresponding local frame of reference is generated and superposed onto the global one, from where the calculation of the hypervolume of interest ensue.

### 1.2 On superior progeny values and short-term genetic gain

As mentioned earlier, plant breeders measure the increase in short-term genetic gain, from one breeding generation to the next, by observing and recording the average weighted phenotypic attributes differential across all individuals and all considered traits making up these two corresponding sequential populations of individuals.

In order to measure this gain, we employ the aggregated so-called superior progeny values of all crossing combinations featuring in a solution. First we assume that the probabilistic crossing combination trait phenotypic values are each normally distributed with means and variances provided as part of the GAPD input data, and that we define an arbitrary percentage target threshold such that any progeny displaying phenotypic trait values produced beyond it is considered to be "superior" (i.e. in the breeders/decision makers' eyes) with respect to those specific traits. The crossing combination trait value delimiters on the axes of the respective normal distributions which define the area under the curve delimited by such percentage target threshold are then defined as the superior progeny values of these distributions (see also Figure 6b).

The aggregated superior progeny value of any given crossing combination is thereby obtained by multiplying each of its normalized superior progeny values by the preferential weight we assign to the corresponding trait, and then by summing up all those terms. In other words, we multiply the vector of the crossing combination's normalized superior progeny values by the transpose of the vector of preconfigured trait preferential weights. The short-term genetic gain objective of any given solution in the domain space of the GAPD problem is then evaluated by summing up the resulting corresponding crossing combinations' superior progeny values featured within this solution.

### 1.3 On cultivar diversity and long-term genetic gain

According little importance to the population diversity factor is the right strategy to adopt only if the time span of a breeding program is that of a few years. But for most breeding programs, while short-term genetic gain is crucial toward the improvement of a cultivar, focusing the bulk of efforts on selecting only crossing combinations that maximize the superior progeny metric value of a solution does not address the genetic diversity element that this solution brings to the breeding population.

Practically speaking, a breeding program adopting a strategy that solely focuses on the genetic performance of a subgroup of elite individuals (that share similar capacities for producing progenies with promising phenotypic improvement, and thereby usually share similar genetic material) maximizes the exploitation of specific sub-regions of the genome, while paying little to no attention to the preservation of the genomic configuration in the remaining regions.

Ignoring the genetic diversity in a cultivar can be undesirable for four main reasons:

1. While big jumps in improvement can be observed across the desirable traits over the course of a few generations, the exploitation of only specific parts of the genome will inevitably converge after a relatively few numbers of generations, following which no further phenotypic performance improvement is possible. This is mainly due to the increasingly limited number of combinations available to configure the DNA of new population progenies.

2. A restricted genetic pool increases the rate of inbreeding, which increases the redistribution of alleles from heterozygous to homozygous states. This can lead to the increased expression of deleterious recessive genes and, consequently, to reduced genetic gain performance altogether.

3. While the measure of what "good performance" represents is unlikely to change too much in the short-term, that is however not a certainty in the long-term. In particular, the trait phenotype characteristics deemed worthy at present, in addition to the respective levels of desirability that we allocate to each of those traits, may very well shift in time as the market evolves and farming strategies adapt thereof.

4. Lastly, a diverse cultivar provides more flexibility to recalibrate breeding strategies in the advent of new or changing environments. More specifically, because phenotypic output is not only dependent on genomic configuration, but also on the environment in which the crop is grown, individuals displaying the highest phenotypic performance with respect to the environments in which they are currently harvested might no longer strive under the influences of new, potentially very different farming regions, or as a result of significant shifts in environmental conditions due to climate change in existing farming regions.

### 1.4 On the implementation of secondary traits

However, due to good leaps in the quality and efficiency of the GAPD search algorithm, it is important to also give importance to the presence of secondary traits in the chosen solutions. Because the short-term genetic gain objective function is constructed on a fusion of multiple primary traits, it would not be wise to dilute the importance of those traits by further incorporating secondary traits inside that function, or to over-complicate the function with so many different elements.

Instead, secondary traits are incorporated as hard constraint in the optimization model formulation, with the option of having the full flexibility to relax these constraints whenever their strictness level too negatively impacts the performance of the algorithm.

## 1.5 On solution sizes

In the GAPD, the solution size simply refers to the number of crossing combinations featuring in a given solution. Solution sizes being flexible in the GAPD, their a priori parametric configuration plays a major role in the optimization procedure. In line with significantly increasing the size of the domain space (in contrast to using a fixed solution size), having multiple solution sizes allows us to uncover more interesting, broader, and better spread non-dominated fronts. We also rightfully observe that larger solutions sizes are heavily correlated to higher solution diversity and operating costs, but inversely correlated to short-term genetic gain.

## 1.6 On the simultaneous optimization of multiple goals

In optimization modelling, one of the biggest area for potential failure evolves around the misinterpretation of the quality assessment of solutions. This is because solution quality criteria do not necessarily adhere to what seems the most logical to the modeler, but can also rely heavily on confusing subjective preferences conjured by decision makers. A good balance between these two paradigms is therefore wise.

The optimization process in the GAPD problem focuses on running multiple so-called short-term genetic gain target trials (simply referred to in these notes as trials), each made up of a unique domain space and feasible domain space (defined by both the chosen trial-dependent subset sizes and the hard constraints induced through the trial-dependent crossing combination population sizes). In each trial, we seek to find solutions in which the SPV, minor allele frequency and inbreeding coefficient metrics are simultaneously optimized. More specifically, each trial returns a non-dominated front in bi-objective space, where the SPV objective is optimized on the one side, and the combined minor allele frequency and inbreeding coefficient sub-objectives are optimized on the other side. Each nondominated front is hence made up of several solutions providing the decision makers with various trade-off alternatives between short-term and long-term genetic gain.

## 2 Mathematical modelling

### 2.1 Subset size upper bound

As mentioned earlier, there exists a total of $\frac{1}{2}N(N-1)$ possible crossing combinations in any GAPD problem, where $N$ is the number of parent individuals. We have also established that (1) each crossing combination ($P_i$, $P_j$) is assigned a meticulously derived population size $\Pi(i,j)_{\overline{g}}$ for each trial $\overline{g}$, and (2) the total number of progenies produced in any solution of a GAPD problem (i.e. the sum of the selected crossing combinations' population sizes) are bounded above by the cost/budget constraint value C. We are therefore able to state that there exists a maximum number of crossing combinations $2 \le \kappa_{\overline{g}} < \frac{1}{2}N(N-1)$ beyond which there exists no feasible solutions (i.e. such that all possible solutions of size $\kappa + 1$ are infeasible).

It is useful to establish this bound for use in the strategic configuration of the GAPD optimization algorithms later on. This can simply be achieved by first sorting the population sizes in ascending order, then by adding the population sizes one at a time from the top until the cost constraint is violated.

Mathematically speaking, if we denote $(\Pi(i,j)_{\overline{g}}]_{(r)}$ to be the ordered rank r of the population size of Crossing Combination ($P_i$, $P_j$) in trial $\overline{g}$, then $\kappa_{\overline{g}}$ is the value for which the inequality

$$C - \sum_{r=1}^{\kappa_{\overline{g}}} \left[\Pi(i,j)_{\overline{g}}\right]_{(r)} < \left[\Pi(i,j)_{\overline{g}}\right]_{(r+1)}$$

hold true.

### 2.2 Input data and parametric configuration

Consider a set of candidate parent individuals $\mathcal{P} = \{P_1,...,P_N\}$ forming the (initial) cultivar population of a GAPD problem, with candidate crossing combinations C = {$c_{12}$, $c_{13}$, ... , $c_{(N-1)N}$}, and define the set of secondary traits S = {$s_1$,..., $s_S$}.

Let $\mathcal{U}_s$ represent the combined set of unique (categorical) secondary traits values assigned to all the crossing combinations in C, where $s \in S$. Moreover, define the Inclusion Set $I$ to force certain crossing combinations into any solution, and inversely define the Exclusion Set $\varepsilon$ to prevent certain crossing combinations from featuring in any solution.

Next, let the parameter $\Pi_{ij} \in \mathbb{N}$ represent the progeny population size of Crossing Combination $c_{ij}$ and let $v_{ij}$ represent the superior progeny value of $c_{ij}$, where $i,j \in \{1,...,N\}$. Due to limited amount of resources, we also define the parameter 6 > 0 as the maximum total number of progenies (i.e. operating costs) produced across all selected crossing combinations.

Thereafter, define the so-called G-Matrix **G** as a symmetrical $N$ by $N$ matrix whose entries $G_{ij}$ depict the level of genetic similarity between parent Individuals $i,j \in \{1,...,N\}$, where a higher value indicates more similarity. Although it might appear counter-intuitive at first, it is good to note that, for practical reasons, the diagonal entries in this matrix can also slightly vary from one another (even though those are typically the highest).

More advanced perhaps, we also define the user parameter $\alpha \geq 0$ to factor in the penalty given to parent individuals that feature multiple times in a given solution, and the user parameter $\beta \geq 1$ to favour solutions that contain a higher number of unique/distinct parents (i.e. the total number of parents used at least once in the solution). In other words solutions containing a lot of unique parents, and few parents that are overused, will tend to be favored in terms of their genetic diversity.

Moving on, define the set of all secondary traits constraint objects $\Lambda = \{h_1,...,h_{|\Lambda|}\}$, and define the linking parameter

$$\lambda_{hs} = \begin{cases} 1, \\ 0, \end{cases}$$

if Secondary Trait Constraint $h$ is associated to Secondary Trait $s$, otherwise, for all $h \in \Lambda$ and all $s \in S$. This parametric configuration of course requires both that $|\Lambda| \geq |S|$, and that

$$\sum_{s \in S} \lambda_{hs} = 1$$

for all $h \in \Lambda$.

Hereafter, let $\theta_h \in \mathcal{U}_s$ represent the value assessed in Constraint $h \in \Lambda$ for the corresponding Secondary Trait $s \in S$, and let $\vartheta_{ijs}$ represent the value assigned to Crossing Combination $c_{ij}$ for Secondary Trait $s \in S$. We then construct the parameter

$$\xi_{hij} = \begin{cases} 1, \\ 0, \end{cases}$$

if $\vartheta_{ijs} = \theta_h$ and $\lambda_{hs} = 1$, otherwise,

Finally let $\xi_h^L \in [0,1)$ *and* $\xi_h^U \in (0,1]$ depict the minimum and maximum proportion (respectively) of crossing combinations in a given solution required to match the value $\theta_h$ in Secondary Trait Constraint $h \in \Lambda$, where of course $\xi_h^L < \xi_h^U$.

### 2.3 Decision variables

In the GAPD problem, the domain space is delimited by the set of decision variables

$$x_{ij} = \begin{cases} 1, \\ 0, \end{cases}$$

if parent Individual i is selected to breed with parent Individual $j$, otherwise where $x_{ij}$ is identical to $x_{ji}$, and

$$y_i = \begin{cases} 1, \\ 0, \end{cases}$$

if parent Individual i is selected at least once in the solution, otherwise

In addition, for ease of representation in the model formulation later on, we derive the solution size as

$$\eta = \sum_{i=1}^{N-1} \sum_{j=i+1}^{N} x_{ij}$$

the total number of unique parents featuring in the solution as

$$\gamma = \sum_{i=1}^{N} y_i$$

and the overall number of times that parent Individual $i \in \mathcal{P}$ employed in the solution as

$$\delta_i = \sum_{j=1}^{N} x_{ij} .$$

### 2.4 Objective functions

### 2.4.1 Short-term genetic gain (Max)

$$\frac{1}{\eta} \sum_{i=1}^{N-1} \sum_{j=i+1}^{N} x_{ij} v_{ij}$$

### 2.4.2 Option 1: Inbreeding coefficient (Min)

In the optimization model you will work with, the diversity component will solely be evaluated by a so-called inbreeding coefficient metric (without much loss of generality with regard to the scope of your thesis). There exist additional metrics that can be combined in the objective function (such as the minor alleles frequency metric) to complement the inbreeding coefficient metric, but we will not consider those at this point.

While there exists many mathematical variations in the literature behind the inbreeding coefficient metric, the described herein standalone version aims to meet the following two conditions/axioms in all conceivable scenarios:

I) If (1) all non-diagonal entries in the $G$-Matrix have the same values, (2) all diagonal entries in the $G$-Matrix have the same values, (3) Solution B contains more unique parent individuals than Solution A, regardless of the number of crossing combinations in either solution (i.e. solution sizes), and (4) $\alpha$ is set to 0 if Solution B has a higher size than Solution A, then Solution B must have a smaller/better inbreeding coefficient score than Solution A. The only exception to this condition occurs when $\beta = 1$, in which case do both solutions have the same score.

II) If Solution B contains more crossing combinations than Solution A, but both solutions share the same set of unique parents, and $\alpha$ is set to 0, then the inbreeding coefficient score of Solution B must be identical to that of Solution A.

We henceforth calculate the inbreeding coefficient of solution x with the function

$$\Psi(x) = \frac{2}{\gamma(\gamma-1)} \sum_{i=1}^{N-1} \sum_{j=i+1}^{N} y_i y_j \, \mathrm{G}_{ij} + \frac{1}{2\gamma^{\beta}} \sum_{i=1}^{N} y_i \left(1 + \alpha(\delta_i - 1)\right) \mathrm{G}_{ii}$$

### 2.4.3 Option 2: Inbreeding coefficient Hybrid (min)

$$\sum_{i=1}^{N-1} \sum_{j=i+1}^{N} x_{ij} \left(0.5\mathrm{G}_{ij} + 0.25\mathrm{G}_{ii} + 0.25\mathrm{G}_{jj}\right)$$

$$\Psi(x) = 0.5 \left[ \frac{2}{\gamma(\gamma-1)} \sum_{i=1}^{N-1} \sum_{j=i+1}^{N} y_i y_j \, \mathrm{G}_{ij} + \frac{1}{2\gamma^{\beta}} \sum_{i=1}^{N} y_i \left(1 + \alpha(\delta_i - 1)\right) \mathrm{G}_{ii} \right] + 0.5 \left[ \frac{1}{\eta} \sum_{i=1}^{N-1} \sum_{j=i+1}^{N} x_{ij} \mathrm{G}_{ij} \right]$$

### 2.4.4 Minor alleles frequencies metric (Max)

Consider again an initial GAPD population of $N$ individuals $\{P_1...,P_N\}$, and recall that $\delta_{im} \in \{0, 1, 2\}$ represents the known diploid value of Individual $i \in \{1, ..., N\}$ at Marker $m \in \{1, ..., N\}$. Although these diploid values are actually part of the raw input data, it is nevertheless useful to mention how they were numerically derived from their original allele values in case only such values are provided in certain GAPD scenarios.

Basically, every marker position of any given individual carries a combination of two alleles, say $\mathbf{A}_1$ and $\mathbf{A}_2$, such that there exists a total of three configurations, namely $(\mathbf{A}_1, \mathbf{A}_1)$, $(\mathbf{A}_1, \mathbf{A}_2)$, (or $(\mathbf{A}_2, \mathbf{A}_1)$), and $(\mathbf{A}_2, \mathbf{A}_2)$. For any given marker, the first step then consists in counting how many $\mathbf{A}_1$ and $\mathbf{A}_2$ alleles are there respectively across the population of initial individuals. We then observe which count is the smaller (if not a tie). This identifies the less frequently occurring allele; i.e. the minor allele.

As such, each individual entry is assigned a numerical score for that marker of either one of $\{0, 1, 2\}$, based on if and how many minor alleles this individual possesses for that particular marker. For example, suppose that the minor allele at Marker m is $A_2$. Then, the minor allele score of Individual i at that marker is assigned a value of $\delta_{im} = 0$ if $m_m = (A_1, A_1)$, $\delta_{im} = 1$, if $m_m = (A_1, A_2)$ or $m_m = (A_2, A_1)$, and $\delta_{im} = 2$ if $m_m = (A_2, A_2)$. The interested reader is referred to the R package BCS. Generics for a full derivation of this procedure.

The so-called minor allele frequency of Marker m is henceforth calculated as

$$\phi_m = \begin{cases} \min\left\{ \dfrac{1}{N} \sum_{i=1}^{N} \delta_{im} \; ; \quad 2 - \left( \dfrac{1}{N} \sum_{i=1}^{N} \delta_{im} \right) \right\}, & \text{if } \dfrac{1}{N} \sum_{i=1}^{N} \delta_{im} \in (0, 2) \\ 1, & \text{otherwise} \end{cases}$$

where it should be noted that $\phi_m$ only takes on extreme values 0 or 2 if (respectively): (1) the entire population carries the same allele for that specific marker (while the other allele does not feature at all), consequently also pointing out its diversity saturation; and (2) the allele types are exactly evenly spread out across all individuals. As seen above, the assigned value to either of these cases is, by default, set to 1 so that, by definition of the logarithmic function, its contribution toward the minor allele frequency long-term genetic gain score will (appropriately) be zero.

We are thereafter interested in evaluating the contribution of minor/rare alleles that each individual packs within itself. Unlike more traditional minor allele contribution metrics, which consider additive linear preference over all possible allele rarity levels (e.g. De Beukelaer 2017).

### 2.4.5 Operating costs (Min)

$$\sum_{i=1}^{N-1} \sum_{j=i+1}^{N} x_{ij}\Pi_{ij}$$

### 2.5 Hard constraints

### 2.5.1 Inclusion set

$$\sum_{c_{ij}\in\mathcal{J}} x_{ij} = |\mathcal{J}|$$

### 2.5.2 Exclusion set

$$\sum_{c_{ij}\in\mathcal{E}} x_{ij} = 0$$

### 2.5.3 Operating costs

$$\sum_{i=1}^{N-1} \sum_{j=i+1}^{N} x_{ij}\Pi_{ij} \leq B$$

### 2.5.4 Secondary traits

$$\xi_h^L \leq \frac{1}{\eta} \sum_{i=1}^{N-1} \sum_{j=i+1}^{N} x_{ij}\xi_{hij} \leq \xi_h^U \quad \forall\, h \in \mathcal{H}$$

### 2.6 Diversity sub-objective metrics bounds

While the diversity objective function may very well be assessed through the original numerical scales whenever either one of the minor alleles frequency or inbreeding coefficient metric is adopted in the model formulation, this is certainly not the case in instances where both these metrics are simultaneously adopted. Clearly this is because the objective space ranges of these metrics (mapped from the search space of a given GAPD scenario) are made up of very different sets of values. When both metrics are employed simultaneously, the diversity score of any given solution can therefore not be assessed by merely adding the two metric scores multiplied by their respective user-defined preferential weights. This would result in one of the sub-objective being significantly favored over the other. And this independently of the assigned preferential weights, which should only be configured in respect of the preference that we give to each sub-objective, never as a means to "fairly" compensate for these variations in sub-objective ranges.

The reason we normalize a metric is hence simply to map the entire spectrum of values spanning a sub-objective range in $\mathbb{R}$ onto the interval [0, 1] or (0, 1), thereby allowing a much "fairer" objective assessment of the two (or more) combined sub-objectives. In order to do this, we first need to find adequate numerical lower and upper bounds for each diversity metric and for each trial. For the minor alleles frequency sub-objective in trial $\overline{g}$, we (ideally) need to find the lower and upper bound values $L[\Phi_{\bar{g}}]$ and $U[\Phi_{\bar{g}}]$ so that

$$\nexists\ \Phi(x) > L\left[\Phi_{\overline{g}}\right]\ \forall\ x \in S_{\overline{g}}$$

(where $S_{\bar{g}}$ is the search space of trial $\bar{g}$), and

$$\nexists\ \Phi(x) > L\left[\Phi_{\overline{g}}\right]\ \forall\ x \in S_{\overline{g}}$$

Similarly, we define $L[\Phi_{\bar{g}}]$ and $U[\Phi_{\bar{g}}]$ as the lower and upper bound values of the inbreeding coefficient sub-objective in trial $\bar{g}$.

### 2.7 Numerical examples

### 2.7.1 Secondary traits constraints

The implementation of secondary traits constraints is perhaps also a bit tricky. Since the topic will strongly evolve around those, It may therefore be useful to clarify the abstract notation in the previous section through the use of a simple hypothetical example.

Consider again a cultivar set $\mathcal{P} = \{P_1, P_2, P_3, P_4\}$ with corresponding crossing combinations set combinations C = $\{c_{12}, c_{13}, c_{14}, c_{23}, c_{24}, c_{34}\}$, and assume that two secondary traits $S = \{s_1, s_2\}$ and secondary traits constraints $\Lambda = \{h_1, h_2, h_3\}$ are in use.

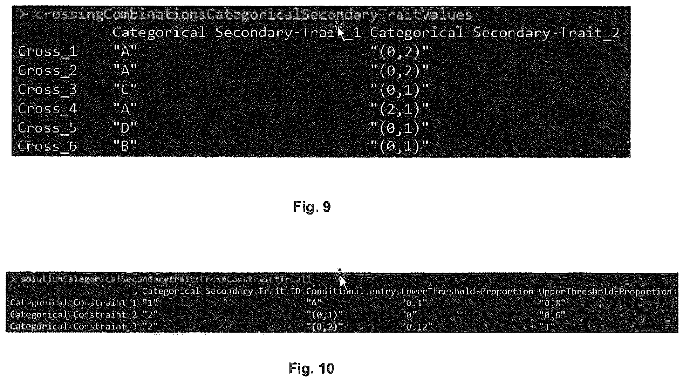

Given the secondary traits constraints input data provided in Figs. 9 and 10, we can then extract the following parameters:

$$\mathcal{U}_1 = \{"A", "B", "C", "D"\} \text{ and } \mathcal{U}_2 = \{"(0,1)", "(0,2)", "(2,1)"\}$$

$$\lambda_{11} = 1, \lambda_{22} = 1, \lambda_{32} = 1 \text{ and } \lambda_{hs} = 0 \text{ for all other pairs of indices}$$

$$\theta_1 = "A", \theta_2 = "(0,1)" \text{ and } \theta_3 = "(0,2)"$$

$$\vartheta_{121} = "A", \vartheta_{131} = "A", \vartheta_{141} = "C", ..., \vartheta_{242} = "(0,1)" \text{ and } \vartheta_{342} = "(0,1)"$$

$$\xi_{112} = 1, \xi_{113} = 1, \xi_{114} = 0, ..., \xi_{324} = 0 \text{ and } \xi_{334} = 0$$

$$\xi_1^L = 0.1, \xi_1^U = 0.8, \xi_2^L = 0, \xi_2^U = 0.6, \xi_3^L = 0.12, \xi_3^U = 1$$

Next, considering the random solution $x = \{\{P_1, P_2\}, \{P_1, P_4\}, \{P_2, P_4\}\} = \{c_{12}, c_{14}, c_{24}\}$ in the domain space of a GAPD problem (where $\eta = 3$). Then, we can see that this solution is only feasible with respect to Secondary Trait Constraints 1 and 3 (i.e. overall infeasible), because

1.

$$0.1 \leq \frac{1}{3}(\xi_{112} + \xi_{114} + \xi_{124}) = \frac{1}{3}(1 + 0 + 0) = 0.333 \leq 0.8,$$

2.

$$0 \leq \frac{1}{3}(\xi_{212} + \xi_{214} + \xi_{224}) = \frac{1}{3}(0 + 1 + 1) = 0.667 > 0.6,$$

3.

$$0.12 \leq \frac{1}{3}(\xi_{312} + \xi_{314} + \xi_{324}) = \frac{1}{3}(1 + 0 + 0) = 0.333 \leq 1$$

(respectively).

## 2.8 Optimization model formulation

Based on everything discussed up to this point, the aim in this GAPD optimization problem is therefore to maximise

$$\frac{1}{\eta} \sum_{i=1}^{N-1} \sum_{j=i+1}^{N} x_{ij} v_{ij}$$

maximise

$$w_\Phi \frac{\Phi(y_1, \ldots, y_N) - L[\Phi_{\bar{g}}]}{U[\Phi_{\bar{g}}] - L[\Phi_{\bar{g}}]} - w_\Psi \frac{\Psi(y_1, \ldots, y_N, x_{12}, \ldots, x_{(N-1)N}) - L[\Psi_{\bar{g}}]}{U[\Psi_{\bar{g}}] - L[\Psi_{\bar{g}}]}$$

minimise

$$\sum_{i=1}^{N-1} \sum_{j=i+1}^{N} x_{ij} \Pi_{ij}$$

such that

$$\sum_{c_{ij} \in \mathcal{J}} x_{ij} = |I|, \qquad\qquad\qquad (1)$$

$$\sum_{c_{ij} \in \mathcal{E}} x_{ij} = 0, \qquad\qquad\qquad (2)$$

$$\sum_{i=1}^{N-1} \sum_{j=i+1}^{N} x_{ij} \Pi_{ij} \leq B, \qquad\qquad\qquad (3)$$

$$\xi_h^L \leq \frac{1}{\eta} \sum_{i=1}^{N-1} \sum_{j=i+1}^{N} x_{ij} \xi_{hij} \leq \xi_h^U, \qquad\qquad h \in \mathrm{H}, \qquad (4)$$

$$2 \leq \eta = \sum_{i=1}^{N-1} \sum_{j=i+1}^{N} x_{ij} < \frac{N(N-1)}{2}, \qquad\qquad (5)$$

$$\gamma = \sum_{i=1}^{N} y_i, \qquad\qquad\qquad (6)$$

$$\eta \geq \delta_i = \sum_{j=1}^{N} x_{ij} \geq y_i \qquad\qquad i \in \mathcal{P}, \qquad (7)$$

$$x_{ij} \equiv x_{ji} \in \{0,1\} \qquad\qquad i,j \in \mathcal{P}, \quad (8)$$

$$x_{ii} = 0 \qquad\qquad\qquad i \in \mathcal{P}, \qquad (9)$$

$$y_i \in \{0,1\} \qquad\qquad i \in \mathcal{P}. \qquad (10)$$

### 3 Post-optimization solution selection

As mentioned earlier, when dealing with multi-objective optimization models, the output of the search algorithm consists in not one, but rather multiple high-quality solutions (this set is referred to in the literature as the non-dominated front). Each of these solutions embody different trade-off compromises across the objective functions. Therefore, no solution is objectively better than any others within that output set. Out of this set, the decision maker is then required to select the solution with the objective function score compromises that best matches his/her preferences.

Because the GAPD framework can be completely automated, however, there can be no human-in-the-loop intervention at any point in the entire framework (i.e. anywhere between entering the raw data objects at the very beginning, to analyzing the returned solution at the very end). This means that we had to develop an approach that allows the framework to pick a single solution from the non-dominated front returned by the search algorithm.

The approach developed by the inventors and described herein is referred to as post-optimization weighted multi-objective scalarization. It combines a method of normalizing the objective function values and multiplying them by numerical weights that are representative of the decision maker's preferences. This approach is not only efficient at selecting the best solution from a GAPD non-dominated front, but also relatively easily implementable into the framework.

### 3.1 Abstract description

Suppose that the non-dominated front is given by the set of solution $N = \{X_1, X_2, ..., X_N\}$, and let the preferential weights of the decision maker be given by the values $\{w_1, w_2, w_3\}$, where each of these weights correspond to the GAPD objective functions depicted in the optimization model in Section 2.8 (namely short-term genetic gain, genetic diversity and operational costs).

Next, let $\lceil O_1 \rceil$ and $\lfloor O_1 \rfloor$ represent the upper and lower bound values of the first objective function of all solutions in $N$. Similarly, let $\lceil O_2 \rceil$ $\lfloor O_2 \rfloor$, $\lceil O_3 \rceil$, and $\lfloor O_3 \rfloor$ respectively represent the upper and lower bound values of the second and third objective functions of all solutions in N. Furthermore, let $O_1(x_i)$, $O_2(x_i)$ and $O_3(x_i)$ represent the first, second, and third objective scores (respectively) of solution $x_i \in N$.

The so-called scalarized objective value of solution $x_i$ is then calculated as

$$\Theta(x_i) = w_1 \left( \frac{O_1(x_i) - \lfloor O_1 \rfloor}{\lceil O_1 \rceil - \lfloor O_1 \rfloor} \right) + w_2 \left( \frac{O_2(x_i) - \lfloor O_2 \rfloor}{\lceil O_2 \rceil - \lfloor O_2 \rfloor} \right) - w_3 \left( \frac{O_3(x_i) - \lfloor O_3 \rfloor}{\lceil O_3 \rceil - \lfloor O_3 \rfloor} \right)$$

where it must be noted that the first two terms are added due to the corresponding objective functions being maximized, while the third term is subtracted as a result of the corresponding objective function being minimised.

Each solution $N$ is then ranked according to their scalarized objective values. The GAPD framework will therefore pick the solution which possess the highest rank.

### 3.2 Numerical example

To illustrate the principles described in the section above, consider a (hypothetical) non-dominated front output of a GAPD optimization process consisting of 5 solutions, along with their respective objective function values, laid out in Table 1.

*Table 1: Hypothetical non-dominated front of a GAPD optimization problem consisting of five solutions, along with their respective objective function values.*

| N | Objective function values | | |
|---|---|---|---|
| | $O_1(x_i)$ | $O_2(x_i)$ | $O_3(x_i)$ |
| $x_1$ | 5 | 10 | 3000 |
| $x_2$ | 4.5 | 17 | 3500 |
| $x_3$ | 6 | 9 | 2500 |
| $x_4$ | 3 | 17 | 2800 |
| $x_5$ | 5 | 15 | 3200 |

Next, suppose that the decision maker's preferences with regard to all 3 objective functions is reflected by means of the weights $w_1 = 2$, $w_2 = 1.6$, $w_3 = 1.1$ (i.e. in this case the decision maker cares the most about short-term genetic gain, and second most about genetic diversity). The scalarized objective value of, for example, Solution $x_1$, is then calculated as

$$\Theta(x_1) = 2\left(\frac{5-3}{6-3}\right) + 1.6\left(\frac{10-9}{17-9}\right) - 1.1\left(\frac{3000-2500}{3500-2500}\right)$$

$$= 2\left(\frac{2}{3}\right) + 1.6\left(\frac{1}{8}\right) - 1.1\left(\frac{500}{1000}\right) = 0.983$$

Similarly, we can derive the scalarized objective values of the other 4 solutions in $N$, as shown in Table 2. The so-called preferential ranks of these solutions are also provided in this table. Looking at the results, it follows that Solution $x_3$ is the best. This solution will therefore be reported back to the decision maker as part of the GAPD framework output.

Table 2: Scalarized objective values and preferential ranks of the non-dominated front solutions.

| $N$ | $\Theta(x_i)$ | Rank $(x_i)$ |
|---|---|---|
| $x_1$ | 0.983 | 5 |
| $x_2$ | 1.5 | 3 |
| $x_3$ | 2 | 1 |
| $x_4$ | 1.27 | 4 |
| $x_5$ | 1.76 | 2 |

**4 Alternative GAPD multi-objective optimization methods**

In the GAPD multi-objective optimization model, we employ an approach that aims to find the Pareto front out of the search space of any given problem instance using a metaheuristic search engine. As discussed previously, we refer to the non-dominated front as an approximation of the Pareto front. The more complex the problem instance, the less likely it is that the non-dominated front will converge exactly to the Pareto front. Nevertheless, we have observed that the optimization approach still produces non-dominated fronts of excellent quality, even when dealing with GAPD problem instances of high-complexity. However, other approaches to solve the optimization problems are also conceivable.

This approach is just one of many that we could employ to solve GAPD multi-objective optimization problems. Examples of other potential techniques include:

Hypervolume Chebyshev scalarization (e.g. https://arxiv.org/pdf/2006.04655.pdf)

Mathematical programming (e.g. https://link.springer.com/journal/10107)

Reactive search Optimization (e.g. https://core.ac.uk/download/pdf/11829614.pdf)

Directed search domain (e.g. https://www.tandfonline.com/doi/abs/10.1080/0305215X.2010.497185)

Branch and bound (e.g. https://www.sciencedirect.com/science/article/abs/pii/S037722171730067X)

Indirect/Self organisation Optimization (e.g. https://www.iosotech.com/pab7.htm)

Ziont-Wallenius method (e.g. https://www.sciencedirect.com/science/article/pii/S0307904X11000849) While the invention has been illustrated and described in detail in the drawings and foregoing description, such illustration and description are to be considered exemplary and not restrictive.

The invention is not limited to the disclosed embodiments. In view of the foregoing description and drawings it will be evident to a person skilled in the art that various modifications may be made within the scope of the invention, as defined by the claims.

**Claims**

1. A computer-implemented method (100, 200) for decision support in selecting crossings for population development, the method comprising:

    receiving (S11) a set of candidate parents, a set of traits, and one or more constraints comprising a resource constraint representative of a maximum number of crossings and/or a maximum progeny population size;
    carrying out a multi-objective optimization (S12),

        wherein carrying out the optimization comprises, constrained by the received one or more constraints, optimizing (S12a) at least a first optimization objective, a second optimization objective, and a third optimization objective, and outputting (S12b) a set of solutions, wherein each solution is a list of crossings of parents of the set of candidate parents,
        wherein the first optimization objective is a short-term genetic gain objective associated with the set of traits and the optimization comprises maximizing the short-term genetic gain,
        wherein the second optimization objective is a long-term genetic gain objective and the optimization comprises maximizing the long-term genetic gain, and
        wherein the third optimization objective is a resource requirements objective associated with a predicted required number of progenies and the optimization comprises minimizing the resource requirements;

    evaluating (S13) a pareto front of the set of solutions and selecting a subset of one or more solutions within the set of solutions based on a result of evaluating the pareto front;
    outputting (S14a), to a user, a representation of the potential crossings, their associated short-term genetic gain and long-term genetic gain, and a representation of the subset of one or more solutions, and receiving (S14b) a user input selecting a solution of the subset of solutions, or
    automatically ranking (S14c) the solutions of the subset of solutions based on an objective function used in the multi-objective optimization and automatically selecting (S14d) the highest-ranked solution;
    providing (S15) the list of crossings of the selected solution and a predicted required number of progenies for each of the crossings comprised in the list of crossings.

2. The method of claim 1, wherein evaluating the pareto front comprises using an algorithm, such as a search algorithm, to select a subset of one or more solutions within the set of solutions.

3. The method of claim 1 or 2, comprising, prior to carrying out the optimization, pre-selecting (S10) a subset of candidate parents among the set of candidate parents based on genomic information, wherein the optimization is carried out only for crossings of the subset of candidate parents,
the method particularly comprising simulating, making use of genomic information, mean and variance of a progeny for every possible crossing, wherein the optimization is carried out only for a subset of crossings for which feasibility for a set of genetic gain targets has been confirmed based on the simulated mean and variance.

4. The method of any of the preceding claims, wherein the short-term genetic gain and/or the long-term genetic gain is determined making use of genomic information.

5. The method of any of the preceding claims, wherein the short-term genetic gain is determined based on, e.g. stochastically, predicted superior progeny values for each of the crossings, in particular wherein the superior progeny value for the respective crossing is an aggregated value aggregated over the set of traits with optional weighting of the traits.

6. The method of any of the preceding claims, wherein the long-term genetic gain is representative of a genetic diversity, wherein the genetic diversity is expressed by a minor allele frequency score and/or an inbreeding coefficient.

7. The method of claim 6, wherein the inbreeding coefficient of a solution depends on at least one of a number of times a parent is used for crossing in the solution, a number of unique parents used for crossing in the solution, and the similarity of the parents of the respective crossing.

8. The method of claim 6 or 7, wherein the long-term genetic gain objective is based on both, the inbreeding coefficient and the minor allele frequency score, optionally the method comprising normalization of the inbreeding coefficient and the minor allele frequency score and combining the normalized inbreeding coefficient and minor allele frequency score into a single optimization objective, or
wherein the long-term genetic gain objective is based on only one of the inbreeding coefficient and the minor allele frequency score.

9. The method of any of the preceding claims, wherein the subset of one or more solutions is a first subset of one or more solutions and wherein the method comprises, determining (S16) prior to selecting a solution of the subset of solutions, particularly in response to a user input and/or by automatic determination based on the optimization objective, whether any of the first subset of solutions meets predetermined criteria and, in case none of the first subset of solutions meets the predetermined criteria:

   a) modifying (S17) the set of candidate parents, the set of traits, and/or at least one of the one or more constraints;
   b) carrying (S18) out the optimization with the modified set of candidate parents, the modified set of traits, and/or the modified constraint(s), the optimization comprising outputting a second set of solutions;
   c) evaluating (S19), particularly using a search algorithm, a pareto front of the second set of solutions and selecting a second subset of one or more solutions within the second set of solutions based on a result of evaluating the pareto front;
   d1) outputting (S20a), to a user, a representation of the potential crossings, their associated short-term genetic gain and long-term genetic gain, and a representation of the second subset of one or more solutions, and receiving (S20b) a user input selecting a solution of the second subset of solutions or of the first and second subset of solutions, or
   d2) automatically ranking (S20c) the solutions of the second subset of solutions based on an objective function used in the multi-objective optimization and automatically selecting (S20d) the highest-ranked solution among the second subset of solutions or among the first and second subset of solutions;
   providing (S15) the list of crossings of the selected solution and a predicted required number of progenies for each of the crossings comprised in the list of crossings.

10. The method of any of the preceding claims, the constraints comprising constraints related to the inclusion and/or exclusion of specific crosses and/or constraints related to achieving a maximum value for one or more predetermined traits, and/or constraints related to achieving a constant value for one or more predetermined traits, and/or constraints related to achieving a minimum value for one or more predetermined traits, and/or constraints related to a predetermined proportion of crosses with a desirable characteristic that should make up the final crossing list.

11. The method of any of the preceding claims, wherein the subset of solutions comprises solutions at or within a predetermined distance from the pareto front of solutions of the multi-objective optimization.

12. The method of any of the preceding claims,

   wherein the representation of the potential crossings and their associated short-term genetic gain and long-term genetic gain comprises representing each crossing within a two-dimensional coordinate system, the coordinate system having a first axis representative of the short-term genetic gain and a second axis representative of a long-term genetic gain, and/or
   wherein the representation of the subset of solutions comprises, for each solution of the subset, an indication of a group of crossings associated with the solutions, particularly the group being represented in the coordinate system such as by heatmaps or shapes enclosing the crossings of the group of crossings or other highlights highlighting crossings of the group of crossings,
   wherein, optionally, the representation of the potential crossings, and optionally their associated diversity, further comprises representing each crossing as a geometric shape, such as a circle, within the coordinate system, the size of the geometric shape being correlated with a first metric of the long-term genetic gain and the second axis value is correlated with a second metric of the long-term genetic gain, in particular wherein the first metric is associated with one of an inbreeding coefficient and a minor allele score, and the second metric is associated with the other one of the inbreeding coefficient and the minor allele score.

13. A method (300) comprising the method (100, 200) of any of the preceding claims, further comprising using (S31) the list of crossings and selecting (S32) and crossing (S33) the parents in the manner indicated in the list for generating a progeny population and/or providing resources for population development based on a/the list of crossings.

14. A system (1) comprising a computing system (1a) configured to carry out the method of any of claims 1 to 12.

15. A computer program product comprising instructions which, when the program is executed by a computer, cause the computer to carry out the method of any of claims 1 to 12.

16. A computer-readable medium comprising instructions which, when the program is executed by a computer, cause the computer to carry out the method of any of claims 1 to 12.

100

S10

S11

S12

S12a    S12b

S13

S14a  S14  S14c

S14b        S14d

S15

Fig. 1

200

```
┌ ─ ─ ─ ─ ─ ─ ─ ─ ─ ─ ─ ─ ┐
╎          S10           ╎
└ ─ ─ ─ ─ ─ ─ ─ ─ ─ ─ ─ ─ ┘
              │
              ▼
┌─────────────────────────┐
│          S11            │
└─────────────────────────┘
              │
              ▼
┌─────────────────────────┐
│          S12            │
│  ┌ ─ ─ ─ ┐   ┌ ─ ─ ─ ┐  │
│  ╎ S12a  ╎   ╎ S12b  ╎  │
│  └ ─ ─ ─ ┘   └ ─ ─ ─ ┘  │
└─────────────────────────┘
              │
              ▼
┌─────────────────────────┐
│          S13            │
└─────────────────────────┘
              │
              ▼
┌─────────────────────────────────┐
│              S16                │
└─────────────────────────────────┘
```

S10

S11

S12

S12a    S12b

S13

S16

S17

S18

S19

S20    S20a S20b S20c S20d

S14    S14a S14b S14c S14d

S15

Fig. 2

300

100    200

S31

S32

S33

S34

Fig. 3

1

1a    1b

1d    1c

Fig. 4

Fig. 5

**Fig. 6a**

**Fig. 6b**

Fig. 7a

**Fig. 7b**

**Fig. 7c**

**Fig. 8**

```
> crossingCombinationsCategoricalSecondaryTraitValues
          Categorical Secondary-Trait_1 Categorical Secondary-Trait_2
Cross_1    "A"                          "(0,2)"
Cross_2    "A"                          "(0,2)"
Cross_3    "C"                          "(0,1)"
Cross_4    "A"                          "(2,1)"
Cross_5    "D"                          "(0,1)"
Cross_6    "B"                          "(0,1)"
```

Fig. 9

```
> solutionCategoricalSecondaryTraitsCrossConstraintTrial1
                      Categorical Secondary Trait ID Conditional entry LowerThreshold-Proportion UpperThreshold-Proportion
Categorical Constraint_1 "1"                              "A"           "0.1"                     "0.8"
Categorical Constraint_2 "2"                              "(0,1)"       "0"                       "0.6"
Categorical Constraint_3 "2"                              "(0,2)"       "0.12"                    "1"
```

Fig. 10

Europäisches
Patentamt

European
Patent Office

Office européen
des brevets

# EUROPEAN SEARCH REPORT

**Application Number**

EP 24 18 3325

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AKDEMIR DENIZ ET AL: "Multi-objective optimized genomic breeding strategies for sustainable food improvement", HEREDITY, SPRINGER INTERNATIONAL PUBLISHING, CHAM, vol. 122, no. 5, 27 September 2018 (2018-09-27), pages 672-683, XP036755150, ISSN: 0018-067X, DOI: 10.1038/S41437-018-0147-1 [retrieved on 2018-09-27] * abstract * * page 674, column 1, paragraph 4 - column 2, paragraph 2; table 1 * * page 675, column 1, paragraph 2 - column 2, paragraph 2; figures 3,4 * * page 676, column 1, paragraph 5 - page 678, column 2, paragraph 2 * ----- | 1-16 | INV. G16B20/00 G16B40/00 |
| A | US 2019/172548 A1 (DUCROCQ SEBASTIEN [FR] ET AL) 6 June 2019 (2019-06-06) * abstract * * n; paragraphs [0006], [0007], [0033] - [0036] * ----- | 1-16 | |
| A | ANTOINE ALLIER ET AL: "Optimized breeding strategies to harness genetic resources with different performance levels", BMC GENOMICS, BIOMED CENTRAL LTD, LONDON, UK, vol. 21, no. 1, 11 May 2020 (2020-05-11), pages 1-16, XP021276252, DOI: 10.1186/S12864-020-6756-0 * abstract * * page 13/16, column 1, paragraph 3 - column 2, paragraph 2 * ----- -/-- | 1-16 | |

**TECHNICAL FIELDS SEARCHED (IPC)**

G16B

The present search report has been drawn up for all claims

| Place of search | Date of completion of the search | Examiner |
|---|---|---|
| Munich | 19 November 2024 | Thumb, Werner |

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 18 3325

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOEINIZADE SABA ET AL: "Optimizing Selection and Mating in Genomic Selection with a Look-Ahead Approach: An Operations Research Framework", G3 GENES - GENOMES - GENETICS, vol. 9, no. 7, 1 July 2019 (2019-07-01), pages 2123-2133, XP093125399, ISSN: 2160-1836, DOI: 10.1534/g3.118.200842 Retrieved from the Internet: URL:https://academic.oup.com/g3journal/article-pdf/9/7/2123/40634123/g3journal2123.pdf> * the whole document * ----- | 1-16 | |
| | | | **TECHNICAL FIELDS SEARCHED (IPC)** |

The present search report has been drawn up for all claims

| Place of search | Date of completion of the search | Examiner |
|---|---|---|
| Munich | 19 November 2024 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................................................
& : member of the same patent family, corresponding document

EPO FORM 1503 03.82 (P04C01)

EP 4 668 276 A1

## ANNEX TO THE EUROPEAN SEARCH REPORT
## ON EUROPEAN PATENT APPLICATION NO.

EP 24 18 3325

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2024

| Patent document cited in search report | Publication date | Patent family member(s) | | Publication date |
|---|---|---|---|---|
| US 2019172548 A1 | 06-06-2019 | EP | 3261007 A1 | 27-12-2017 |
| | | EP | 3475862 A2 | 01-05-2019 |
| | | US | 2019172548 A1 | 06-06-2019 |
| | | WO | 2017220640 A2 | 28-12-2017 |

EPO FORM P0459

For more details about this annex : see Official Journal of the European Patent Office, No. 12/82

39

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHENGQIANG ZHONG** ; **JEAN-LUC JANNINK**. *Genetics*, 2007, vol. 177, 567-576

- **DENIZ AKDEMIR** ; **JULIO I. SANCHEZ**. *Efficient Breeding by Genomic Mating, Frontiers in Genetics*, 2016, vol. 7, 210